# EUROPÄISCHE PATENTSCHRIFT

(11) **EP 3 769 071 B1**
(45) Veröffentlichungstag und Bekanntmachung des Hinweises auf die Patenterteilung: **01.01.2025**
(21) Anmeldenummer: 19709750.4
(22) Anmeldetag: 15.03.2019
(51) Int. Cl.: G01N 21/35, G01N 30/00

(54) **VERFAHREN ZUR UNTERSUCHUNG VON PROZESSSTRÖMEN**
METHOD FOR ANALYSING PROCESS STREAMS
PROCÉDÉ D'ANALYSE DE FLUX DE TRAITEMENT

(30) Priorität: 20.03.2018 EP 18162722
(43) Veröffentlichungstag der Anmeldung: 27.01.2021
(73) Patentinhaber: HTE GmbH The High Throughput Experimentation Company, 69123 Heidelberg (DE)
(72) Erfinder: KIRCHMANN, Marius, 69123 Heidelberg (DE); HAUBER, Christoph, 69123 Heidelberg (DE)
(74) Vertreter: BASF IP Association
(86) Internationale Anmeldenummer: PCT/EP2019/056537
(87) Internationale Veröffentlichungsnummer: WO 2019/179887

(56) Entgegenhaltungen:
- WO-A1-2016/110408
- WO-A1-2017/075140
- JP-A- H0 232 253
- JP-A- H0 232 253
- US-A- 5 349 188
- US-A1- 2007 082 407
- US-A1- 2007 082 407
- US-A1- 2015 260 695
- US-A1- 2015 260 695

## Beschreibung

Die vorliegende Erfindung betrifft ein Verfahren zur Untersuchung von Prozessströmen, deren Zusammensetzung sich in kurzen Zeitabständen ändert. Das erfindungsgemäße Verfahren ist flexibel und vielseitig einsetzbar. Ein bevorzugtes Einsatzgebiet des Verfahrens betrifft katalytische Prozesse mit Produktströmen mit einer Vielzahl von Komponenten, insbesondere mit fünf oder mehr verschiedenen kohlenwasserstoffhaltigen Komponenten.

Die vorliegend interessierenden katalytischen Prozesse sind dadurch gekennzeichnet, dass sie im industriellen Produktionsbetrieb und in Strömungsreaktoren durchgeführt werden. Bei zahlreichen Prozessen bilden sich in den Katalysatorbetten der Reaktoren komplexe Reaktionsnetzwerke aus. Der Begriff "komplexe Reaktionsnetzwerke" im Sinne der vorliegenden Erfindung bedeutet, dass sich die in den Reaktoren ablaufenden Reaktionen nicht mehr durch einfache chemische Reaktionsmechanismen, beispielsweise eine Kinetik erster Ordnung beschreiben lassen, und eine Vielzahl von Reaktionen gleichzeitig im Katalysatorbett stattfinden. Im Ergebnis werden Prozessströme erhalten, die eine Zusammensetzung mit einer Vielzahl von unterschiedlichen Komponenten aufweisen.

Die Komplexität, die mit einer Charakterisierung dieser Prozessströme einhergeht, resultiert auch daraus, dass sich die Zusammensetzungen der Prozessströme in vergleichsweise kurzen Zeiträumen innerhalb von Stunden, Minuten und Sekunden ändern können. Ursachen für diese Änderungen sind beispielsweise Deaktivierungs- oder Einlaufverhalten der Katalysatoren, Änderungen in der Zusammensetzung von Edukten oder Änderungen von Prozessbedingungen.

Im Stand der Technik sind online-spektroskopische Verfahren zur Charakterisierung von komplexen Probengemischen mittels chemometrischer Verfahren bei industriellen Produktionsprozessen prinzipiell bekannt. Nachfolgend wird eine kurze Übersicht zum Stand der Technik gegeben.

Im Bereich der Petrochemie und bei der Abmischung von Kraftstoffen werden seit mehreren Jahrzehnten spektroskopische Analyseverfahren zur Bestimmung der Zusammensetzung von Prozessströmen eingesetzt. Die Kraftstoffe können mehrere hundert unterschiedliche Verbindungen enthalten und die Zielzusammensetzung der Kraftstoffe muss in einem sehr engen Bereich liegen, damit diese die gewünschten Eigenschaften in Bezug auf die Klopffestigkeit liefern. Da in den Raffinerien große Mengen an Kraftstoffen produziert werden, eignen sich die spektroskopischen Verfahren in besonderer Weise, um in kurzen Zeitabständen die chemische Zusammensetzung der Ströme zu ermitteln. Als beispielhaft für die Abmischung von oxygenathaltigen Kohlenwasserstoffen sei die US 5,596,196 genannt, die offenbart, dass oxygenathaltige Kohlenwasserstoffe unter Verwendung von Raman-Spektroskopie und multivariater Analyse mit hoher Genauigkeit bestimmt werden können. Die resultierenden Messsignale können dazu verwendet werden, um die Konzentration von den Komponenten in den Produkten zu kontrollieren. Dieses Verfahren wird mithilfe einer Glasfaseroptik direkt im Prozessstrom in der Flüssigphase durchgeführt und kann dabei in weniger als einer Minute Messwerte liefern.

In der PCT-Anmeldung WO 01/02088 A1 wird ein Verfahren zur Kontrolle des Produktionsprozesses von Polyhydroxyalkoholen beschrieben. In diesem Verfahren wird die Zusammensetzung des flüssigen Prozessstroms kontinuierlich mittels IR-Spektroskopie überwacht. Anschließend wird die Differenz zwischen der gemessenen und der gewünschten Zusammensetzung bestimmt, um die Parameter des Herstellungsprozesses zu kontrollieren. Bei dem Prozess handelt es sich um ein mehrstufiges Verfahren. Messungen können an unterschiedlichen Punkten des Prozessstroms durchgeführt werden. Mittels IR-Spektrometer wird die Absorbanz des Prozessstroms gemessen, anschließend bestimmt ein Computer die Zusammensetzung des Prozessstroms in Übereinstimmung mit einem zuvor programmierten Kalibrierungsmodell.

Im Zusammenhang mit Abmischungen von flüssigen Prozessströmen ist auch das US-Patent US 5,349,188 von Ashland Oil Inc. zu nennen, das die NIR-Analyse von PIANO-Konstituenten und die Bestimmung der Oktanzahl von Kohlenwasserstoffen behandelt. Die PIANO-Konstituenten enthalten die Gruppen Paraffine, iso-Paraffine, Aromaten, Naphthene und Olefine. Bei dem Verfahren werden die Absorptionsbanden gemessen, die für die Gruppe der einzelnen PIANO-Konstituenten charakteristisch sind.

Weiterhin wurde von Ashland Oil Inc. in WO 99/02973 ein Prozess / Apparatur zur Untersuchung von Kohlenwasserstoffen mittels NIR offenbart, wobei Konzentrationen von Einzelkomponenten im aromatischen Bereich (BTX) mit spezifischen Absorptionsbanden im NIR-Spektrum mithilfe statistischer Methoden korreliert und dadurch eine Messung sowie Prozesssteuerung in der Flüssigphase bei Raffinerieprozessen ermöglicht wird.

In der US 5,360 972 von der Western Atlas International Inc. wird die Verbesserung von IR-spektroskopischen Messungen an Materialen offenbart, die dadurch erreicht wird, dass die die spektroskopischen Daten mit Kalibrierungsmodellen analysiert werden. Es werden unterschiedliche mathematische Kalibrierungsmodelle beschrieben. In Verbindung mit der Durchführung der Untersuchungen wird beschrieben, dass Messungen an Standard-Proben vorgenommen werden, wobei hierbei ein NEG Calibration Set verwendet wird. Bei den Standardproben handelt es sich um flüssige Proben, die mit einem GC-Messgerät durchgeführt wurden, das unabhängig von einem Prozessstrom betrieben wird.

Zusammenfassend lässt sich feststellen, dass im Stand der Technik bevorzugt in der flüssigen Phase und in großtechnischen Anlagen gearbeitet wird. Für die Kalibrierung der spektroskopischen Methode (MIR, NIR, Raman) werden Proben offline, also außerhalb des eigentlichen Reaktionsablaufes und der Testbedingungen, mithilfe von Gas- oder Flüssigchromatographie analysiert und dann ebenfalls spektroskopisch vermessen. Auf Basis dieser offline-Kalibrierung wird mithilfe statistischer Methoden ein Modell entwickelt und die spektroskopische Analyse nachfolgend online, also unter den eigentlichen Reaktionsbedingungen, im Prozessstrom betrieben.

In der US 2007/0082407 A1 von Joseph P. Little wird die Durchführung einer online-Analyse eines Prozessstromes beschrieben und beansprucht, die auf der Verwendung eines optischen Analysators basiert. Der optische Analysator umfasst eine Anzahl von optischen Sensoren, die zur Durchführung von spektroskopischen Analysen verwendet werden können. Mittels der spektroskopischen Analysen wird die chemische Zusammensetzung von Erdgas bestimmt. Die Sensoren sind in ein Datensammelsystem eingebunden, um die Zusammensetzung von Erdgas an unterschiedlichen Standorten innerhalb eines Speicher- und Übertragungsinfrastruktur in einer nahezu kontinuierlichen Weise zu bestimmen.

In der JPH0232253 von Shimadzu Corp. wird eine Vorrichtung beschrieben, die die Verarbeitung von Daten ermöglicht, wobei die Detektorsignale von Chromatograph und Spektrometer Gegenstand von einer Echtzeitdatenverarbeitung sind.

In der US 2015/0260695 A1 von Prism Analytical Tech. Inc. wird ein Messgerät beschrieben und beansprucht, das aus einer Kombination von Gaschromatograph und FTIR-Spektrometer besteht. Das FTIR-Spektrometer ist der Trennsäule des Gaschromatographen nachgeschaltet. Bei der Verwendung des Gerätes wird die Probe zunächst in die einzelnen Komponenten aufgetrennt und die einzelnen Komponenten werden einer IR-Messzelle zugeführt.

Auch die WO 2017/075140 A1 stammt von der gleichen Anmelderin wie die US 2015/0260695 A1, das heißt von der Firma Prism Analytical Tech. Inc., und beschreibt und beansprucht ein Messgerät, dass aus der Kombination von Gaschromatograph und FTIR-spektroskopischer Analyse besteht.

Die US 2002/0031737 A1 von der American Air Liquid Inc. beschreibt ein Verfahren und eine Apparatur zu Verwendung einer durchstimmbaren Laserdiode, um spezielle Komponenten in einem Gasfluss aus einer Prozessleitung zu untersuchen, wobei die Untersuchungen in Echtzeit an Verbrennungsprozessen durchgeführt werden. Die Untersuchungen beziehen sich auf die Spezies O₂, CO, H₂O. Der Anwendungsbereich ist somit auf einige wenige Komponenten beschränkt.

In einer bevorzugten Ausführungsform wird das erfindungsgemäße Verfahren auf dem Gebiet der Hochdurchsatzforschung eingesetzt. Das Gebiet der Hochdurchsatzforschung betrifft die Beschleunigung und die Parallelisierung von Forschungsexperimenten. Von besonders großer Bedeutung sind die Verbesserung von katalytischen Prozessen und Katalysatoren mittels Hochdurchsatzforschung, da die Verbesserungen dazu beitragen, Energie einzusparen und/oder Resourcen zu schonen.

Als Stand der Technik auf dem Gebiet der Hochdurchsatzforschung sind beispielhaft die US-Anmeldung US 2002/0182735 A1 von Kibby et al. oder auch die PCT-Anmeldung WO 2016/110408 A1 von Kirchmann et al. zu nennen. In der US 2002/0182735 A1 wird die Verwendung von Mikroreaktoren in der kombinatorischen Chemie beschrieben. Die bei den Reaktionen entstehenden Produkte können in Bezug auf unterschiedliche Eigenschaften untersucht und geprüft werden, wobei diese Eigenschaften auch die Oktan- und Cetanzahl einschließen können. Nachdem die Reaktion erfolgt ist, können die Reaktionsprodukte individuell von einem Kanal zu einem analytischen Messgerät transferiert werden, insbesondere zu einem Chromatographen.

Die PCT-Anmeldung WO 2016/110408 A1 von Kirchmann et al., die vom gleichen Anmelder stammt wie die vorliegende Anmeldung, betrifft eine Apparatur und ein Verfahren zur Untersuchung von Naphtha-Reformierungsprozessen. Die Apparatur weist eine Vielzahl von parallel angeordneten Reaktoren auf. Die Ausgangsleitungen der Reaktoren befinden sich in einer Verbindung mit einem online-Gaschromatographen. Anhand von der gaschromatographischen Analyse der Produktfluidströme werden die Reaktionsprozesse optimiert, die in den einzelnen parallel angeordneten Reaktoren ablaufen.

Eine erfindungsgemäße Aufgabe ist es, ein Verfahren zur Untersuchung von Prozessströmen bereitzustellen, insbesondere von Prozessströmen, deren Zusammensetzung sich zeitlich, insbesondere auch über kürzere Zeiträume ändern kann. Mittels der erfindungsgemäßen Verfahren sollte es auch möglich sein, Änderungen der Zusammensetzung der Prozessströme zu überwachen, die in den oben genannten kurzen Zeitabständen auftreten. Eine weitere Aufgabe ist es, ein Verfahren bereitzustellen, welches vorzugsweise in der Hochdurchsatzforschung eingesetzt werden kann, wobei mittels des Verfahrens eine Mehrzahl von Prozessen, die in einer parallelen Anordnung durchgeführt werden, untersucht werden können. Weiterhin soll ein Verfahren zum Untersuchen von Katalysatoren, insbesondere zum parallelen Untersuchen von Katalysatoren unter möglichst praxisnahen Reaktionsbedingungen bereitgestellt werden.

Diese und andere Aufgaben werden gelöst durch ein Verfahren zur Untersuchung von zumindest einem Prozessstrom, der zumindest fünf verschiedene kohlenwasserstoffhaltige Komponenten umfasst, wobei das Verfahren zumindest die folgenden Schritte umfasst:
a) Bereitstellen von zumindest einer Prozessstromleitung (35), welche in Wirkverbindung mit zumindest einem online-IR-Spektrometer (2) sowie in Wirkverbindung mit zumindest einem online-Gaschromatographen (1) ist,
b) Durchleiten von zumindest einem Prozessstrom durch die zumindest eine Prozessstromleitung (35), wobei während dieses Durchleitens des Prozessstroms durch die Prozessstromleitung (35) eine analytische Charakterisierung des Prozessstroms mittels online-IR-Spektrometer (2) und online-Gaschromatograph (1) durchgeführt wird.

Vorzugsweise erfolgt Schritt b) innerhalb eines Zeitfensters, das kleiner als 1 Stunde ist, vorzugsweise kleiner als 40 Minuten.

Im Rahmen des erfindungsgemäßen Verfahrens werden die Vorteile von Chromatographie (hohe Detailtiefe) und Spektroskopie (Schnelligkeit) kombiniert. Dabei werden beide online, also während der katalytischen Umsetzung, entweder parallel oder sequentiell mit bekanntem Zeitversatz, unmittelbar im Produktstrom eingesetzt. Ein vorteilhafter Aspekt des erfindungsgemäßen Verfahrens ist der Einsatz einer online-gaschromatographischen Methode, da die online-Gaschromatographie es ermöglicht, eine Auftrennung der Reaktionsprodukte in die einzelnen chemischen Verbindungen vorzunehmen und eine detaillierte qualitative und quantitative Analyse liefert. Die gaschromatographische Analyse liefert dabei die relevante Information zur chemischen Zusammensetzung, und zwar anhand von Einzelkomponenten und der Menge an den Einzelkomponenten. Anhand dieser Information lassen sich die Zielparameter ableiten.

Diese Zielparameter sind vorzugsweise Parameter aus der Gruppe Konversion, Oktanzahl, Selektivität ausgewählt.

Die Chromatographie hat jedoch auch die Eigenschaft, dass sie diese Ergebnisse mit einem Zeitversatz liefert, welcher insbesondere bei komplexen Prozessströmen im Bereich von 20 bis 180 min liegen kann und zur Erfassung von schnellen Änderungen der Produktströme gegebenenfalls zu langsam ist.

Demgegenüber liefern spektroskopische Verfahren Informationen in Form von Absorption in Abhängigkeit von der Wellenzahl, wobei jedes Spektrum charakteristische Banden liefert, die zu einer bestimmten Stoffkonzentration und Zeit gehören. Der Vorteil der spektroskopischen Methoden ist, dass die Ergebnisse quasi simultan vorliegen und somit angenähert eine kontinuierliche Analyse möglich ist. Die Messzeit der online-spektroskopischen Analyse kann im Sekundenbereich liegen. Somit ermöglicht das erfindungsgemäße Verfahren, dass eine zeitliche Auflösung im Bereich von Sekunden erreicht werden kann.

Ein spektroskopisches Verfahren kann somit zum schnellen Regeln benutzt werden, wobei beim Erreichen eines Zielparameters und zum Aufnehmen von Messdaten mit hoher Informationsdichte wieder chromatographische Verfahren eingesetzt werden, um mehr Details zu erhalten.

In der Regel ist die Verwendung einer spektroskopischen Analysenmethode mit einem hohen Aufwand bei der Kalibrierung verbunden. Insbesondere erfordert die Untersuchung von komplexen Prozessströmen eine Kalibrierung unter Verwendung eines chemometrischen Modells.

Ein Vorteil des erfindungsgemäßen Verfahrens ist, dass das Verfahren unter Prozessbedingungen durchgeführt werden kann, die den Prozessbedingungen von realen (also tatsächlich bei der Durchführung der Reaktion im großtechnischen Maßstab auftretenden Reaktionsbedingungen) Prozessströmen entsprechen.

Vorzugsweise werden mittels des erfindungsgemäßen Verfahrens Prozesse untersucht, welche in einem Zeitfenster von 5 sec - 30 Tagen, weiter vorzugsweise im Zeitfenster von 30 sec - 14

Tagen, darüber hinaus bevorzugt 60 sec - 7 Tagen ablaufen, und welche mit sonst im Technikums-Maßstab gebräuchlichen chromatographischen Methoden zeitlich nicht mehr verfolgt werden können, oder bei denen der Einsatz von Kontrollschleifen zur Nachregelung nicht mehr schnell genug erfolgen kann, um Änderungen im Produktspektrum zu verfolgen.

Es ist jedoch auch möglich, dass erfindungsgemäße Verfahren in Verbindung mit der Untersuchung von Prozessströmen einzusetzen, die über längere Zeiträume als 30 Tage vorliegen, beispielsweise kann das Verfahren auch in Verbindung mit Prozessströmen durchgeführt werden, bei denen das Zeitfenster im Bereich von 2 - 24 Monaten liegt.

Das erfindungsgemäße Verfahren bietet den Vorteil, dass die Durchführung der GC-Messmethode und die Durchführung der IR-spektroskopischen Messmethode in-situ, d.h. unmittelbar im Prozessstrom, durchgeführt werden. Vorzugsweise werden die Kalibrierung der GC-Messmethode und die Kalibrierung der IR-spektroskopischen Messmethode sowie die Korrelation beider Messmethoden auf der Basis der in-situ-Messungen durchgeführt. Darüber hinaus bevorzugt wird das Verfahren als ein selbstlernendes System betrieben. In dieser bevorzugten Ausführungsform des selbstlernenden Systems ist das Verfahren dadurch gekennzeichnet, dass das Verfahren ohne Probenanalysen durchgeführt wird, die außerhalb des Prozessstroms analysiert werden.

Es ist darauf hinzuweisen, dass das erfindungsgemäße Verfahren die Kombination von unterschiedlichen analytischen Methoden umfasst, die mit dem Prozessstrom in direkter Wirkverbindung bestehen. Die resultierenden Analysen basieren auf nahezu identischen Proben des Prozessstromes. Dadurch resultieren Synergieeffekte was die Sensitivität des erfindungsgemäßen Verfahrens betrifft. Weitere technische Vorteile sind mit der Rückkopplung verbunden, die dadurch gegeben ist, dass mittels der Daten die Prozesssteuerung betrieben werden kann.

Demgegenüber sind diejenigen Geräte, die als offline-Geräte verwendet werden, unabhängig von der Prozessstromleitung und weisen auch keine Verbindung zum Prozessstrom auf. Daher ist es mittels derjenigen Geräte, die als offline-Gerät, so zu sagen in einem ex situ Verfahren bzw. außerhalb des Prozessstroms, betrieben werden, nicht möglich, den Prozessstrom durch eine in-situ-Methode zu analysieren.

In einer bevorzugten Ausführungsform sind die Analyseneinheiten, online-Gaschromatograph (1) und das online-IR-Spektrometer (2), mit einer gemeinsamen Prozesssteuereinheit (4) gekoppelt sind, wobei die Prozesssteuereinheit mit einem Prozessraum (11) in Wirkverbindung steht und den im Prozessraum ablaufenden Prozess kontrolliert, steuert oder regelt.

Vorzugsweise wird das Verfahren zur Untersuchung von Prozessströmen durchgeführt, bei denen die Anzahl an kohlenwasserstoffhaltigen Komponenten in den einzelnen Prozessströmen ≥ 5 ist, weiter vorzugsweise ist die Anzahl der kohlenwasserstoffhaltigen Komponenten in den einzelnen Prozessströmen ≥ 5 bis 300. Noch weiter vorzugsweise ist die Anzahl der kohlenwasserstoffhaltigen Komponenten in den einzelnen Prozessströmen ≥ 10 bis 250.

In einer bevorzugten Ausführungsform handelt es sich um ein Verfahren, welches des Weiteren die folgenden Schritte umfasst:
c) Auswerten der bei der analytischen Charakterisierung des Prozessstroms mittels online-IR-Spektrometer erhaltenen Spektral-Daten als Funktion eines Durchführungszeitpunkts dieser spektroskopischen Analyse des Prozessstroms,
d) Auswerten der bei der analytischen Charakterisierung des Prozessstroms mittels des online-Gaschromatographen erhaltenen Chromatographie-Daten als Funktion einer Probenentnahmezeit für dabei aus dem Prozessstrom entnommene Proben,
e) Auf maschinellem Lernen beruhendes Trainieren eines Modells, das eine mathematischen Beziehung zwischen Spektral-Daten und dazu korrespondierenden Chromatographie-Daten bezüglich eines selben Prozessstroms modelliert, mittels der in den Schritten c) und d) erhaltenen Auswertungsergebnisse bezüglich des in Schritt b) durch die Prozessstromleitung geleiteten Prozessstroms.

Es ist bevorzugt, dass das Training des Modells sozusagen in-situ - während der Reaktion oder während des Prozesses - stattfindet. Dies hat den Vorteil, dass eine externe Kalibrierung, die aufwändig sein kann, unnötig ist. Das Training des Modells findet also in-situ während der Reaktion statt, und eine aufwändige externe Kalibrierung ist nicht nötig. Das Modell kann für jeden neuen Prozessstrom angepasst und ergänzt werden oder es kann ein neues Modell trainiert werden und das Risiko von Überanpassung wird minimiert.

Ein solcher Trainingsdatensatz wird bevorzugt folgendermaßen erhalten:
1) Bei schneller Änderung des Prozessstromes < 30 min: Durchführung der Reaktion bei vergleichsweise "milden" Prozessbedingungen, welche die Geschwindigkeit der Änderung zwar verlangsamen, aber trotzdem repräsentative Produktzusammensetzungen liefern (niedrige WHSV, Temperatur,...). Gegebenenfalls auch zusätzliche Variation der Prozessparameter, um einen breiteren Bereich von Produktzusammensetzungen zu gewinnen.
2) Bei langsamer Änderung des Prozessstromes > 30 min: Prinzipiell wäre es möglich, bei langsamem Ablauf der Reaktion ausschließlich mit dem GC zu arbeiten, eine schnelle Analytik bietet jedoch Möglichkeiten, den Parallelisierungsgrad zu erhöhen bzw. verschiedene Produktströme zu erfassen bzw. beim Regeln auf einen Zielparameter, diesen schneller zu erreichen.

In Ausführungsformen des erfindungsgemäßen Verfahrens kann nach Erhalt des Trainingsdatensatzes in Schritt e) beziehungsweise gem. der Schritte c) - e) das zur Bestimmung der Produktzusammensetzung eingesetzte chromatographische Verfahren pausiert werden, und die Messung erfolgt ausschließlich über das spektroskopische Verfahren. (Gaschromatographie kann jedoch zur punktuellen Kontrolle eingesetzt werden).

Während einer Trainingsphase werden durch die Analyseergebnisse repräsentative Bereiche von Zusammensetzungen des Prozessstromes gesammelt, diese können durch Variation von Prozessparametern wie Temperatur, Druck, WHSV oder der Eduktzusammensetzung ergänzt werden.

Im Fall (zu) schneller zeitlicher Veränderung des Prozessstromes, welches eine ungenügende zeitlicher Auflösung für gaschromatographische Analyseverfahren ergeben würde, kann durch geeignete Variation von Prozessparametern die Deaktivierung verlangsamt werden (z.B. durch Variation von WHSV, T, H₂ Partialdruck, Wasserdampfaddition etc.).

Es ist eine Ausführungsform des Verfahrens besonders bevorzugt, in welcher, vorzugsweise während der Trainingsphase, die Schritte c) - e) durchgeführt werden und zumindest ein Reaktionsparameter gegenüber dem selben Reaktionsparameter, wie dieser in den Schritten a) und/oder b) eingestellt wird, verändert ist, wobei dieser Parameter bevorzugt ausgewählt ist aus: WHSV, Temperatur, Gesamtdruck und/oder Partialdruck von Reaktanden. Durch die gezielte Veränderung der Reaktionsparameter lässt sich die Reaktionsgeschwindigkeit beziehungsweise der Umsatz vermindern beziehungsweise erhöhen.

Bei diesen Reaktionsparametern oder Prozessparametern handelt es sich vorzugsweise um einen oder mehrere Parameter, die ausgewählt werden aus der Gruppe WHSV, T, Gesamtdruck, Partialdrücke der Reaktanden, Zugabe von Ko-Feedmolekülen, beispielsweise Wasser als Ko-Feedmolekül. Durch die Charakterisierung des Prozessstroms, der bei einem Satz von Prozessparametern erhalten wird, wird jeweils ein Datensatz mit analytischen Charakterisierungsdaten erhalten, der für den jeweiligen Parameterraum charakteristisch ist.

Es ist bevorzugt, dass die Prozessparameter in einem Prozessparameterraum liegen, der für das jeweils untersuchte Verfahren von technischer Bedeutung ist.

Vorzugsweise liegt die Temperatur des Prozesses, das mit dem erfindungsgemäßen Verfahren gekoppelt ist, in einem Bereich von 50 - 1000 °C, weiter vorzugsweise in einem Bereich von 150 - 750 °C, darüber hinaus bevorzugt in einem Bereich von 250 - 650 °C.

Der Druck des Prozesses liegt vorzugweise in einem Bereich von 0,5 - 500 bar, weiter vorzugsweise in einem Bereich von 1 - 250 bar, darüber hinaus bevorzugt in einem Bereich von 5 - 200 bar. Die vorliegende Angabe des Drucks bezieht sich auf den absoluten Druckwert beziehungsweise (d.h. p.a.).

Die WHSV liegt vorzugweise in einem Bereich von 0.01 - 500 h⁻¹, weiter vorzugsweise in einem Bereich von 0.5- 20 h⁻¹, darüber hinaus bevorzugt in einem Bereich von 1 - 5 h⁻¹.

In einer bevorzugten Ausführungsform ist das erfindungsgemäße Verfahren dadurch gekennzeichnet, dass es zumindest zwei unterschiedliche Phasen umfasst, wobei es sich bei der einen

Phase um eine die Schritte a) bis e) umfassende Trainingsphase und bei der zweiten Phase um eine eigentliche Messphase handelt, in der eine analytische Charakterisierung eines durch die zumindest eine Prozessstromleitung geleiteten Prozessstroms mittels des online-IR-Spektrometers (2) auf Basis des in der Trainingsphase trainierten Modells erfolgt.

In einer bevorzugten Ausführungsform wird bei dem erfindungsgemäßen Verfahren zwischen Trainingsphase und Messphase unterschieden. In der Messphase liegen alle Reaktionsparameter typischerweise in einem Zielparameterraum. In der Trainingsphase dagegen liegen die Reaktionsparameter typischerweise noch nicht im Zielparameterraum beziehungsweise wird zumindest ein Reaktionsparameter in einer kontrollierten Art und Weise variiert, um einen repräsentativen Bereich von unterschiedlichen Prozessströmen mit unterschiedlichen Zusammensetzungen zu generieren.

Bei dem erfindungsgemäßen Verfahren ist es auch bevorzugt, dass es sich bei dem durch die Prozessstromleitung geführten Prozessstrom um einen gasförmigen Prozessstrom handelt, vorzugsweise ist die Temperatur des gasförmigen Prozessstroms im Bereich von 20 - 350 °C, weiter vorzugsweise im Bereich von 50 - 220 °C. Die Temperierung der Prozessstromleitung stellt sicher, dass die kohlenwasserstoffhaltigen Komponenten im Prozessstrom in der Gasphase vorliegen. Dadurch kann die Zeit eingespart werden, die für eine Auskondensation der kohlenwasserstoffhaltigen Komponenten erforderlich ist. In bevorzugten Ausführungsbeispielen wird die Temperatur so eingestellt, dass ein Signal-zu-Rauschverhältnis erhalten wird, in welchem die Kopplungen von Schwingungsbanden reduziert sind.

Im Fall, dass der online-Gaschromatograph (1) und das online-IR-Spektrometer (2) seriell im Hinblick auf die Prozessstromleitung angeordnet sind, ist es bevorzugt, dass die Messungen mit einem zeitlichen Versatz durchgeführt werden, wobei der zeitliche Versatz im Bereich von 1 sec bis 180 sec liegt.

Bevorzugt ist eine Ausführungsform des erfindungsgemäßen Verfahrens, in welcher das Verfahren eine Rückkopplung umfasst. In einer bevorzugten Ausführungsform ist das Verfahren dadurch gekennzeichnet, dass die Analyseneinheiten, online-Gaschromatograph (1) und das online-IR-Spektrometer (2), mit einer gemeinsamen Prozesssteuereinheit (4) gekoppelt sind, wobei die Prozesssteuereinheit (4) mit einem Prozessraum (11) in Wirkverbindung steht und den im Prozessraum ablaufenden Prozess kontrolliert, steuert oder regelt. Die ermittelten Messdaten werden dazu benutzt, den Prozess zu steuern oder zu regeln beziehungsweise auch zu kontrollieren. Die kurzen Messzeiten sind dabei von besonderem Vorteil, da hierdurch eine schnelle Rückkopplung erfolgt, die im Bereich der Zeitdauer der online-IR-Messung liegt. Der Grund ist, dass die Zeitdauer der Prozessierung der Daten und der Weitergabe von Regel- oder Steuersignalen an Prozesseinheiten sehr kurz ist und nur Millisekunden beträgt.

Bevorzugt ist, dass die Prozesssteuereinheit (4) den Prozess so regelt, dass mittels der Anpassung eines Prozessbetriebsparameters die Produktstruktur kontrolliert wird, vorzugsweise wird die Produktstruktur in einer Weise kontrolliert, dass die Oktanzahl konstant ist, dass die Selektivität konstant ist oder dass die Konversion konstant ist, weiter vorzugweise wird die Regelung durch die Änderung der Parameter aus der Gruppe Temperatur und WHSV vorgenommen.

Das Verfahren zur Untersuchung von zumindest einem Prozessstrom wird vorzugsweise zur Untersuchung von einem Prozessstrom eingesetzt, der aus der Gruppe ausgewählt aus Methanol-Konversionssprozessen wie MTO (z.B. Methanol-to-Olefins), Dehydrierreaktionen (z.B. Propandehydrogenierungen), Kopplungsreaktionen (z.B. Methankopplungen), Naphtha Reforming und Prozessen zur Synthese und Umwandlung von Aromaten (z.B. Transalkylierungen, Alkylierungen, Dealkylierungen) stammt.

Bei dem Verfahren zur Untersuchung von zumindest einem Prozessstrom wird als online-IR-Spektrometer (2) vorzugsweise ein Gerät eingesetzt, das im mittleren IR-Bereich (MIR) arbeitet und bei dem die Wellenzahlen im Bereich von 400 cm⁻¹ - 3500 cm⁻¹ liegen.

Bei dem erfindungsgemäßen Verfahren zur Untersuchung von zumindest einem Prozessstrom ist es auch bevorzugt, dass das Modell oder die Methode, wie im Schritts e) eingesetzt, eine statistische Methode umfasst, die ausgewählt ist aus der Gruppe multivariater Analysen wie Hauptkomponentenanalyse (PCA), partieller Regression kleinster Quadrate (PLS), Hauptkomponentenregression (PCR), multilinearer Regressionsanalyse (MLR), Diskriminanzanalyse oder neuronaler Netze. Eine Korrelation zwischen den erzielten Analysenergebnissen, die mittels der online-IR-Spektroskopie und der online-Gaschromatographie erzielt werden, erfolgt dadurch, dass die Analysenergebnisse durch geeignete statistische Verfahren miteinander in Beziehung gesetzt werden und ein Modell trainiert wird. Geeignete statische Verfahren sind beispielsweise diejenigen, die aus der Gruppe multivariate Analysen wie Hauptkomponentenanalyse (PCA), partieller Regression kleinster Quadrate (PLS), Hauptkomponentenregression (PCR), multilinearer Regressionsanalyse (MLR), Diskriminanzanalyse oder neuronaler Netze ausgewählt sind.

Bei dem erfindungsgemäßen Verfahren zur Untersuchung von zumindest einem Prozessstrom ist es weiterhin bevorzugt, dass zusätzliche Daten zum Training des Modells, welche gemäß den Schritten c) bis e) vorgesehen sind, durch eine Variation von Prozessparametern wie der Temperatur, des Drucks, des Partialdrucks der Edukte oder der WHSV erhalten werden.

In einer bevorzugten Ausführungsform betrifft das Verfahren zur Untersuchung von zumindest einem Prozessstrom, einen aus einem katalytischen Prozess stammenden Prozessstrom, bei dem innerhalb des Prozessraums ein Feststoffkatalysator angeordnet ist, wobei die Masse des innerhalb des Prozessraums angeordneten Feststoffkatalysators im Bereich 0,1-200 ccm, vorzugsweise zwischen 0,2 - 20 ccm, liegt.

Die vorliegende Erfindung betrifft auch die Verwendung des erfindungsgemäßen Verfahrens in den unterschiedlichen Ausführungsformen, die im Rahmen der vorliegenden Beschreibung dargestellt sind, zur Hochdurchsatztestung von zumindest vier, vorzugsweise zumindest acht, weiter vorzugsweise zumindest zwölf, in parallelen Reaktoren angeordneten Katalysatoren.

Beim Betrieb von Hochdurchsatzapparaturen ist die Verwendung von leistungsfähigen analytischen Methoden von Interesse, um die Reaktionsprodukte qualitativ und quantitativ zu erfassen. In einer bevorzugten Ausführungsform wird das erfindungsgemäße Verfahren in der Hochdurchsatztestung und in Verbindung mit der Untersuchung von Katalysatoren eingesetzt. Hierbei ist es auch bevorzugt, dass die Katalysatoren in parallel angeordneten Prozessräumen vorliegen.

Vorzugsweise handelt es sich bei den Prozessräumen um Reaktoren und vorzugsweise sind in den Reaktoren Katalysatoren angeordnet. Vorzugsweise wird das erfindungsgemäße Verfahren zur Untersuchung von Prozessströmen durchgeführt, bei denen die Prozessstromleitung (35) mit vier parallel angeordneten Reaktoren in Wirkverbindung steht. Weiter vorzugsweise steht die Prozessstromleitung (35) mit zumindest acht parallel angeordneten Reaktoren in Wirkverbindung, weiter vorzugsweise zumindest zwölf parallel angeordneten Reaktoren und darüber hinaus bevorzugt mit sechzehn parallel angeordneten Reaktoren. Vorzugsweise handelt es sich bei den Reaktoren um Rohrreaktoren.

Die Kombination des erfindungsgemäßen Verfahrens in Verbindung mit Hochdurchsatzforschung und Hochdurchsatzapparaturen stellt eine Ausführungsform dar, die bevorzugt ist, da sich Synergieeffekte ergeben, beispielsweise dergestalt, dass während der Trainingsphase oder der Kalibrierphase aufgezeichnete Datensätze in kürzeren Zeiten und mit verbesserter Effizienz eingesetzt werden können. Dabei liefern die parallel angeordnete Prozessräume, vorzugsweise Reaktoren, die mit unterschiedlichen Katalysatoren beladen sind, Prozessströme, die Unterschiede aufweisen und die für die Kalibrierung verwendet werden können. Die parallele Durchführung ermöglicht es, dass die Kalibrierphase in das Messverfahren integriert werden kann.

Die Verwendung des erfindungsgemäßen Verfahrens ist in Verbindung mit einer Hochdurchsatzapparatur besonders vorteilhaft, da das Verfahren eine große Flexibilität aufweist, was den chemischen Prozess betrifft und sich die Hochdurchsatzapparatur in einfacher Weise umrüsten lässt, wenn diese von einem chemischen Reaktionsprozess auf einen anderen chemischen Reaktionsprozess umgerüstet werden muss, was insbesondere im Forschungsbetrieb häufig der Fall ist.

Mittels des erfindungsgemäßen Verfahrens können Reaktionsnetzwerke und Prozesse mit verbesserter Datentiefe untersucht und Mechanismen der Katalysatordeaktivierung besser verstanden werden, was möglicherweise die Grundlage zur Entwicklung von besseren Katalysatoren bildet und dadurch zur Einsparung von Energie und Ressourcen führen könnte.

In einer bevorzugten Ausführungsform umfasst das erfindungsgemäße Verfahren zur Untersuchung von Prozessströmen zusätzlich auch einen oder mehrere Prozessschritte zur Regenerierung von deaktiviertem Katalysator.

Bei der Regenerierung werden vorzugsweise Ablagerungen entfernt, die sich auf dem Katalysator abgeschieden haben. Zur Entfernung der Ablagerung kann beispielsweise die Temperatur des Katalysators erhöht werden oder die Ablagerungen können in Gegenwart eines sauerstoffhaltigen Gasstroms abgebrannt werden. Bei Untersuchungen zum Koksabbrand kann die Analyse gezielt auf die Produktkomponenten CO, CO₂ und H₂O gerichtet werden.

In einer bevorzugten Ausführungsform wird das Verfahren zur Untersuchung von Reaktionskinetiken eingesetzt. Dabei ist es weiter bevorzugt, das Verfahren in Verbindung mit Prozessen einzusetzen, die kurze Kontaktzeiten oder kurze Deaktivierungszeiten aufweisen.

In bevorzugten Ausführungsformen beträgt die IR-Messzeit pro Messpunkt ≤ 10 sec, ≤ 5 sec, ≤ 2,5 sec, ≤ 1,25 sec. Der Begriff IR-Messzeit bezieht sich auf die gesamte Messdauer und beinhaltet, die Zeit für das Scannen sowie auch die Zeit für die Datenauswertung.

In Ausführungsformen, insbesondere für Kinetik-Untersuchungen, liegt die IR-Messzeit pro Messpunkt im Bereich von Zehntelsekunden, wobei zunächst nur Scans aufgezeichnet werden, die im Anschluss auf die Aufzeichnung mathematisch verarbeitet werden.

Die Zeitdauer der IR-Messzeit stellt eine Größe dar, welche für die Zeitauflösung des erfindungsgemäßen Verfahrens in Bezug auf die Detektion von Veränderungen in der Zusammensetzung des Prozessstroms und dem damit verbundenen Prozess charakteristisch ist.

Das erfindungsgemäße Verfahren eignet sich zur Untersuchung von Langzeitprozessen oder Kurzzeitprozessen. Ein vorteilhafter Aspekt des erfindungsgemäßen Verfahrens ist, dass es zeitlich in flexibler Weise eingesetzt werden kann: Entweder kann das erfindungsgemäße Verfahren zur Kontrolle und Überwachung von Langzeitprozessen oder Industrieanlagen verwendet werden, oder es kann zur Durchführung von Kurzzeitexperimenten verwendet werden. Die Langzeitprozesse liegen vorzugweise in einem Zeitbereich von 2 Woche - 2 Jahren. Die Kurzzeitprozesse liegen vorzugsweise im Bereich von 2 Minuten bis 300 Stunden.

Kurzzeitprozesse sind vorzugsweise dadurch gekennzeichnet, dass innerhalb von 1 - 100 Minuten, weiter vorzugsweise in einem Zeitbereich von 1,5 - 60 Minuten, darüber hinaus in einem Zeitbereich von 2 - 30 Minuten, eine Deaktivierung des Katalysators erfolgt. Daraus resultiert der vorteilhafte Aspekt, dass die Trainingsphase und die Messphase zu einer gemeinsamen Trainings- und Messphase kombiniert werden. Dieser vorteilhafte Aspekt von Kurzeituntersuchungen ist insbesondere in Kombination mit Regenerierungsprozessen von technischer Bedeutung und von großem Nutzen. Die Kombination mit Regenerierungsprozessen ist dadurch gegeben, dass das Verfahren zunächst einen Konversionsprozess umfasst, dem dann ein Regenerierungsprozess folgt. Vorzugsweise wird die Abfolge aus Konversionsprozess und Regenerierungsprozess wiederholt, wobei es sich bei der Wiederholung um eine zyklische Betriebsweise handelt. Vorzugweise ist die Anzahl der Wiederholungen beziehungsweise Zyklen ≥ 2, weiter vorzugsweise ist die Anzahl der Zyklen ≥ 5, noch weiter vorzugsweise ist die Anzahl der Zyklen ≥ 10.

Die Trainingsphase und die Messphase unterscheiden sich dahingehend, dass die Prozessparameter während der Messphase im Zielparameterraum liegen. Bei der Durchführung der Trainingsphase ist es bevorzugt, dass zumindest ein Prozessparameter eine signifikante Abweichung aufweist und nicht im Zielparameterraum liegt.

In einer bevorzugten Ausführungsform bezieht sich das erfindungsgemäße Verfahren auf ein Verfahren zur Hochdurchsatztestung von einer Vielzahl von Katalysatoren, die in parallel angeordneten Prozessräumen vorliegen. Vorzugsweise handelt es sich bei den parallel angeordneten Prozessräumen um parallel angeordnete Reaktoren. In dieser bevorzugten Ausführungsform ist es denkbar, dass die Abweichung des Prozessparameters dadurch bewerkstelligt wird, dass das Verfahren mit parallel angeordneten Reaktoren durchgeführt wird, wobei die Variation des Prozessparameters dadurch erfolgt, dass die parallel angeordneten Reaktoren mit unterschiedlichen Katalysatormaterialien und/oder mit unterschiedlichen Mengen an Katalysatormaterial befüllt werden. Somit kann bei dieser bevorzugten Ausführungsform des Verfahrens die Abweichung oder die Variation des Prozessparameters innerhalb des Systems erzeugt werden. Die Durchführung des Verfahrens und die darin enthaltene Kalibrierung während der Trainingsphase werden auch am experimentellen Beispiel A.2 illustriert, das sich auf die Veredelung von Aromatengemischen bezieht.

Das in Beispiel A.2 beschriebene Verfahren wurde mit vier unterschiedlichen Katalysatoren durchgeführt, wobei der Konversionsprozess zunächst so durchgeführt wurde, dass alle vier unterschiedlichen Katalysatoren bei der gleichen Temperatur gelagert wurden. Die Kalibriermessungen wurden dadurch weiter vertieft, dass auch noch Messungen bei unterschiedlichen Temperaturen durchgeführt wurden. Somit wurde der Prozessparameter Katalysator und der Prozessparameter Temperatur variiert, um ausreichende Daten während der Trainingsphase zu sammeln.

Somit ist es bevorzugt, dass während der Trainingsphase bei Vorliegen einzelner ausgewählter Prozessparameterräume sowohl GC-Charakterisierungen als auch IR-Charakterisierungen des Prozessstroms vorgenommen werden. Die Dauer der GC-Methode richtet sich unter anderem nach der Komplexität der Prozessstromzusammensetzung und der gewünschten Zielsetzung bei der Auflösung in Bezug auf die Einzelkomponenten. Somit wird die Dauer der Trainingsphase entscheidend durch die Dauer der GC-Methode bestimmt.

Vorzugsweise werden während der Trainingsphase 4 oder mehr Chromatogramme aufgenommen, weiter vorzugsweise ist die Anzahl der Chromatogramme während der Trainingsphase ≥ 8, noch weiter vorzugsweise ist die Anzahl der Chromatogramme während der Trainingsphase ≥ 15.

Eine weitere Größe, die für das erfindungsgemäße Verfahren charakteristisch ist, ist das Verhältnis an IR-Messungen in Bezug auf GC-Messungen gegeben. Vorzugsweise ist das Verhältnis an IR-Messungen in Bezug auf GC-Messungen ≥ 6, weiter vorzugsweise ist das Verhältnis an IR-Messungen in Bezug auf GC-Messungen ≥ 60, noch weiter vorzugsweise ist das Verhältnis an IR-Messungen in Bezug auf GC-Messungen ≥ 90, darüber hinaus bevorzugt ist das Verhältnis an IR-Messungen in Bezug auf GC-Messungen ≥ 120.

### Beispiele

### A.1 Umsetzung von Methanol zu Olefinen

Zur Illustration des erfindungsgemäßen Verfahrens wurden Untersuchungen zur katalytischen Umsetzung von Methanol zu Olefinen durchgeführt. Die Untersuchungen wurden mittels einer Hochdurchsatzapparatur zur Testung von Katalysatoren durchgeführt, die mit bis zu sechzehn parallel angeordneten Reaktoren bestückt werden konnte.

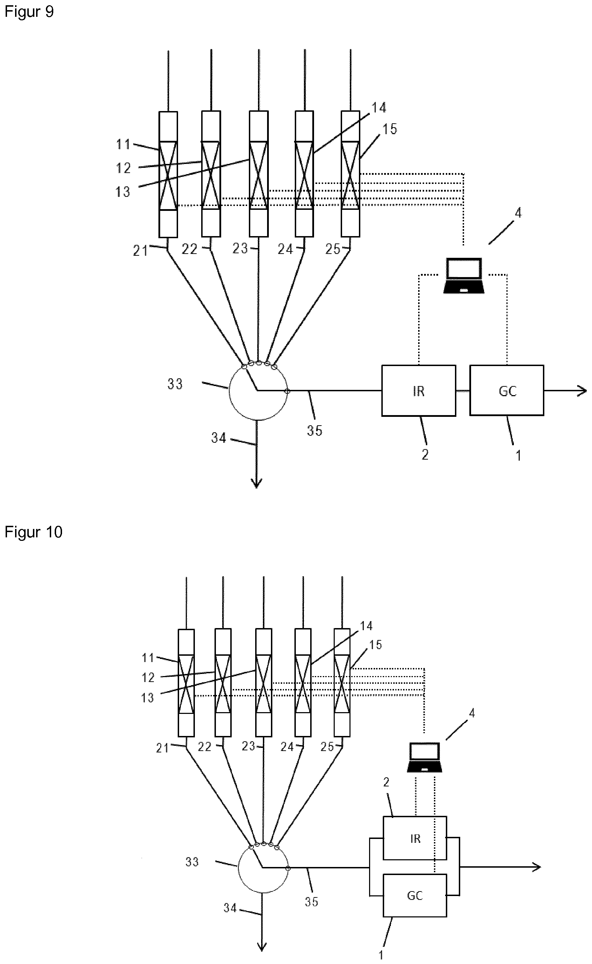

Der schematische Aufbau der Apparatur ist in Figur 9 gezeigt, wobei die in der Darstellung gezeigte Apparatur fünf parallel angeordnete Reaktoren (11) - (15) aufweist. Die Ausgangsleitungen (21) - (25) der Reaktoren sind mit einem Multiportventil (33) verbunden, das zu einer Prozessstromleitung (35) führt, die mit einem online-IR-Spektrometer (2) und einem online-Gaschromatographen (1) in Wirkverbindung steht. Sowohl das online-IR-Spektrometer (2) als auch der online-Gaschromatograph (1) sind mit der Prozesssteuerung der Vorrichtung verbunden. Bei der Testung wurden zwei unterschiedliche zeolithhaltige Katalysatorproben eingesetzt, die kommerziell erhältlich sind und die im Rahmen der Beschreibung mit Probe C1 und Probe C2 bezeichnet werden. In der Figur 9 ist zu erkennen, dass die Prozesssteuereinheit (4), die mit dem online-IR (2) und dem online-GC (1) verbunden ist, je eine Wirkverbindung zu den einzelnen Reaktionsräumen (11) - (15) aufweist. Nicht zu erkennen ist die Wirkverbindung der Prozesssteuereinheit (4) zur Reaktandenzufuhr, die vorzugsweise auch mit der Prozesssteuereinheit (4) verbunden ist.

Die Probe C1 enthielt einen Katalysator auf der Basis von SAPO-34 und die Probe C2 enthielt einen Katalysator auf der Basis von ZSM-5. Zur Vorbereitung der katalytischen Untersuchungen wurden die einzelnen Katalysatorproben jeweils mit Quarzpulver gemischt und in Form von Pulverschüttungen in Rohrreaktoren eingebracht. Die hierbei eingesetzte Menge an Katalysatorproben waren 0,92 g oder 1,85 g. Somit wurden insgesamt vier katalysatorhaltige Probenmischungen vorbereitet. Für Vergleichszwecke wurden zwei Reaktoren mit Quarzpulver als Inertmaterial beladen, welches frei von Katalysator war.

Die verwendeten Reaktoren wiesen eine Rohrlänge von 30 cm und einen Innendurchmesser von 15 mm auf.

Als online-Gaschromatograph (1) wurde ein Hewlett Packard HP 5890 Chromatograph eingesetzt, der mit einer fused-silica-Säule (mit einer Länge von 20 Metern), und FI-Detektor ausgerüstet war. Als online-IR Spektrometer (2) wurde ein FTIR-Spektrometer von der Firma BRU-KER eingesetzt, das für den MIR-Spektralbereich in einem Wellenzahlbereich von 7000 - 400 cm⁻¹ optimiert war. Die Optik des Geräts wurde mit einem He-Ne-Laser gesteuert. Der Strahlengang des Spektrometers war mit einer beheizbaren Gasmesszelle ausgestattet, die ein Innenvolumen von 500 mL und eine optische Strahlenlänge von 75 cm aufwies. Während der Untersuchungen wurde die Messzelle wurde bei einer Temperatur von 180 °C temperiert.

Bei der Zufuhr des methanolhaltigen Eduktstroms wurde zwischen den folgenden zwei Dosiervorgängen unterschieden:
Dosiervorgang 1. kontinuierliche (quasi-kontinuierlich) Dosierung des methanolhaltigen Trägergasstroms während einer Zeitdauer im Bereich von 3 - 30 min und
Dosiervorgang 2. pulsweise Dosierung des methanolhaltigen Trägergasstroms während einer Zeitdauer im Bereich von 5 - 60 Sekunden.

Nach Beendigung der Zuführung des Eduktstroms wurde dem Reaktionsraum ein sauerstoffhaltiger Gasstrom zugeführt, um den entstandenen Koks abzubrennen und den Katalysator zu regenerieren.

Die Durchführung des Verfahrens zur Umsetzung des methanolhaltigen Trägergasstroms erfolgte gemäß dem erfindungsgemäßen Verfahren. Zunächst wurde eine Trainingsphase durchgeführt. Während dieser Trainingsphase wurden die Reaktorräume nacheinander drei unterschiedlichen Edukgastströmen ausgesetzt, die jeweils einen geringeren Eduktgehalt aufwiesen als der Eduktgasstrom, der für den Zielparameterraum vorgesehen war. Um den Eduktgehalt im Eduktgasstrom einzustellen, wurde die Gaslast variiert, die durch die WHSV (weight hourly space velocities) definiert wird. Die für die Trainingsphase gewählte Gaslasten waren durch die folgenden drei Werte der WHSV gekennzeichnet: 0,2 h⁻¹, 0,3 h⁻¹, 0,4 h⁻¹.

Während der unterschiedlichen Dosiervorgänge, die während der Trainingsphase durchgeführt wurden, wurden sowohl gaschromatographische Analysen mittels online-Gaschromatograph (1) als auch spektroskopische Analysen mittels online-Spektrometer (2) an den einzelnen Prozessströmen durchgeführt, die sukzessive aus den Reaktionsräumen über das Multiportventil (33) in die Prozessgasleitung (35) ausgeleitet wurden.

Die einzelnen Analysenergebnisse, die mittels der unterschiedlichen Methoden - d.h. mittels Spektroskopie und mittels Gaschromatographie erzielt worden waren - in Abhängigkeit von Zeit und experimentellen Versuchsparametern, wurden durch mathematische Modelle in Beziehung gesetzt. Beispielsweise wurde anhand der GC-Analysen die jeweilige Menge an Einzelsubstanzen im Prozessstrom quantitativ bestimmt (beispielsweise den Gehalt an Methanol, Dimethylether, Methan, Ethan, Ethen, ...) beziehungsweise wurde auch die Menge an Substanzgruppen innerhalb des Prozessstroms bestimmt (beispielsweise der Gehalt an Aromaten, Olefine, ...).

Zur Quantifizierung wurde im vorliegenden Fall für 9 Einzelsubstanzen ein eigenes Modell verwendet, das auf der Methode der PLS (partial least squares) basiert, wobei 15 Komponenten die Varianz ausreichend erklärt haben. Die Wellenzahlen wurden auf die Bereiche 423-1040 cm⁻¹ sowie 1244-2704 cm⁻¹ reduziert, da insbesondere der Bereich zwischen 1040-1244 cm⁻¹ sowie 2700-3100 cm⁻¹ im verwendeten Konzentrationsbereich an die Absorptionsgrenze gestoßen ist, die eine weitere Datenauswertung verhinderte. Eine feinere oder genauere Abstimmung der optischen Pfadlänge auf den Konzentrationsbereich sollte dazu führen, dass auch diese Bereiche der Wellenzahl in das Auswertungsmodell integriert werden können.

Im Anschluss an die Trainingsphase wurden Untersuchungen im Rahmen der Produktionsphase durchgeführt, die in Gegenwart von einem Eduktgasstrom mit einer - im Vergleich zu der Trainingsphase - nunmehr höheren Gaslast erfolgten, und zwar unter Bedingungen, die im Zielparameterraum lagen.

Vorliegend waren die Untersuchungen während der Produktionsphase beziehungsweise Messphase durch eine Gaslast gekennzeichnet, bei denen die WHSV`s im Bereich von 2 - 20 h⁻¹ lag, wobei die analytische Charakterisierung des Prozessstroms (beziehungsweise der Prozessströme) mittels online-IR-Spektrometer (2) (vorliegend optimiert für den MIR-Bereich). Es wurden im Zeitabstand von 5 Sekunden Messsignale aufgenommen, um den Prozessstrom zu charakterisieren. Die quantitative Auswertung der Bandenbereiche unter Verwendung des Modells, das während der Trainingsphase erstellt wurde, hat es erlaubt, dass anhand der Spektren Rückschlüsse über die Konzentration an Einzelsubstanzen getroffen werden konnten. Somit konnten mittels trainierten Modells anhand der IR-Spektren die Konzentration der Einzelsubstanzen vorhergesagt werden.

Zur Kontrolle der Daten wurden während der Messphase mittels des Gaschromatographen (1) weiterhin in Zeitabständen von jeweils 20 Minuten analytische Charakterisierungen des Prozessstroms parallel zu den IR-Messungen durchgeführt. Bei der Durchführung von sogenannten Pulsexperimenten beziehungsweise Pulsdosierungen wurde auf die zusätzliche Durchführung von gaschromatographischen Analysen ganz verzichtet. Eine Übersicht über die durchgeführten Versuche ist in der Tabelle 1 gezeigt. Drei Experimente wurden während der Trainingsphase und fünf Experimente wurden während der Messphase durchgeführt.

Bei der Umsetzung von Methanol zu Olefinen wurde die Gaslast während der Trainingsphase so gewählt, dass die Werte der WHSV bei 0,2 h⁻¹, 0,3 h⁻¹ und 0,4 h⁻¹ lagen. Während der Messphase lagen die Werte der WHSV bei 5 h⁻¹, 8 h⁻¹, 12 h⁻¹, 16 h⁻¹ und 20 h⁻¹. Somit wurden während der Trainingsphase die Prozessströme generiert und diese dann auch in Abhängigkeit von der Zeit charakterisiert, die für die drei verschiedenen Parameterräume und gewählten Prozessparametern charakteristisch sind. Ob diese drei verschiedenen Parameterräume ausreichend sind, hängt unter anderem vom betrachteten Prozess und dem zeitlichen Verhalten des betrachteten Prozesses ab.

Es ist in Verbindung mit dem erfindungsgemäßen Verfahren bevorzugt, dass während der Trainingsphase eine Anzahl von Prozessparameterräumen untersucht wird, die vorzugsweise ≥ 2 ist. Weiter vorzugweise ist die Anzahl von Prozessparameterräumen, die bei der Durchführung der Trainingsphase zur Generierung und Charakterisierung von Prozessströmen eingesetzt werden ≥ 3, darüber hinaus bevorzugt ist die Anzahl der Prozessparameterräume ≥ 4.

Bei der Durchführung der Messphase liegt der Parameterraum der WHSV vorzugsweise im Bereich von > 1 h⁻¹ bis 20 h⁻¹. Hingegen liegt während der Trainingsphase der Parameterraum der WHSV vorzugsweise im Bereich von 0,1 h⁻¹ bis 1 h⁻¹. Während der Durchführung der Messphase kann der verwendete WHSV-Bereich auch als Zielparameterraum betrachtet werden.

Im Beispiel A.1 liegt die WHSV während der Trainingsphase in signifikanter Weise außerhalb des Zielparameterraums.

In Bezug auf die untere Grenze des Zielparameterraums (d.h. eine WHSV von 5 h⁻¹) während der Messphase) ist die Abweichung des Prozessparameters WHSV während der Trainingsphase nur 8 % des Wertes gegenüber der Messphase. Daraus resultiert die Forderung, dass die Prozessparameter, beziehungsweise zumindest ein Prozessparameter, während der Trainingsphase außerhalb des Zielparameterraums liegen. Dabei bedeutet der Begriff "außerhalb des Zielparameterraums", dass - bei einem Vergleich von Trainingsphase und Messphase - die Abweichung von mindestens einem Prozessparameter ≥ 10 % ist, vorzugsweise ist die Abweichung ≥ 20 %, darüber hinaus bevorzugt ist die Abweichung ≥ 50 %, insbesondere bevorzugt ist eine Abweichung von ≥ 75 %.

In Bezug auf das A.1 war die untere Grenze der WHSV im Zielparameterraum durch eine WHSV von 5 h⁻¹ gegeben. Die WHSV während der Trainingsphase lag bei 0,4 h⁻¹. Somit war die Abweichung bei 4,6 h⁻¹, was einer prozentualen Abweichung von 92 % entspricht. In Bezug auf die obere Grenze des Zielparameters ist die Abweichung durch eine WHSV-Differenz von 19,6 h⁻¹ gegeben, was einer prozentualen Abweichung von 98 % entspricht.

**In Tabelle 1 ist eine Übersicht über die Versuchsnummern und die damit verbundenen Prozessparameter dargestellt.**

| Versuchsnummer | WHSV [h⁻¹] | Temp. [°C] | Betriebsweise | Phase |
|---|---|---|---|---|
| C1_01 | 0,2 | 400 | kontinuierlich | Trainingsphase |
| C1_02 | 0,3 | 400 | kontinuierlich | Trainingsphase |
| C1_03 | 0,4 | 400 | kontinuierlich | Trainingsphase |
| C1_04 | 5 | 400 | kontinuierlich | Messphase |
| C1_05 | 8 | 400 | kontinuierlich | Messphase |
| C1_06 | 12 | 400 | kontinuierlich | Messphase |
| C1_07 | 16 | 400 | kontinuierlich | Messphase |
| C1_08 | 20 | 400 | kontinuierlich | Messphase |

**Tabelle 2 zeigt den Prozentsatz der durch das Modell abgedeckten Varianz in Abhängigkeit der Komponenten (Faktoren).**

| Komponenten | Ethen | Propen | Butene | Methan | Ethan | Propan | Butan | Methanol | DME |
|---|---|---|---|---|---|---|---|---|---|
| 1 | 0,405 | 7,72 | 59,059 | 28,49 | 89,47 | 52,08 | 47,89 | 22,06 | 70,82 |
| 2 | 75,92 | 92,01 | 79,86 | 28,91 | 89,49 | 89,06 | 86,22 | 22,22 | 91,81 |
| 3 | 94,5 | 94,05 | 96,05 | 40,55 | 89,95 | 97,31 | 96,56 | 25,23 | 96,66 |
| 4 | 94,78 | 97,52 | 98,9 | 94,36 | 89,96 | 98,69 | 97,86 | 36,79 | 99,22 |
| 5 | 96 | 98,65 | 99,24 | 95,75 | 90,27 | 98,86 | 97,88 | 94,43 | 99,23 |
| 6 | 98,94 | 99,09 | 99,5 | 96,08 | 97,36 | 99,08 | 99,03 | 95,55 | 99,43 |
| 7 | 99,12 | 99,11 | 99,67 | 98,06 | 97,38 | 99,09 | 99,38 | 95,58 | 99,66 |
| 8 | 99,13 | 99,19 | 99,72 | 98,18 | 97,88 | 99,53 | 99,47 | 95,65 | 99,66 |
| 9 | 99,13 | 99,32 | 99,73 | 98,42 | 98,7 | 99,53 | 99,47 | 95,67 | 99,72 |
| 10 | 99,13 | 99,34 | 99,75 | 98,65 | 98,79 | 99,62 | 99,55 | 95,67 | 99,73 |
| 11 | 99,17 | 99,35 | 99,75 | 98,69 | 98,84 | 99,69 | 99,55 | 95,8 | 99,73 |
| 12 | 99,17 | 99,37 | 99,77 | 98,89 | 99,33 | 99,71 | 99,63 | 95,8 | 99,73 |
| 13 | 99,17 | 99,38 | 99,78 | 98,89 | 99,34 | 99,71 | 99,65 | 95,8 | 99,73 |
| 14 | 99,19 | 99,38 | 99,78 | 98,89 | 99,34 | 99,71 | 99,65 | 95,83 | 99,73 |
| 15 | 99,22 | 99,4 | 99,78 | 98,89 | 99,34 | 99,72 | 99,7 | 95,98 | 99,73 |

### A.2 Verfahren zur Veredelung von Aromatengemischen

Zur Illustration des erfindungsgemäßen Verfahrens wurden auch katalytische Untersuchungen zur Veredelung von Aromatengemischen durchgeführt. Für die Vergleichbarkeit von Katalysatoren ist es in vielen Fällen von Interesse, diese unter den Prozessbedingungen zu betreiben, die so gewählt werden, dass die unterschiedlichen Katalysatoren und Prozesse zu gleichen Zielparametern führen. Bei zahlreichen Prozessen stellt die Konversion diesen Zielparameter dar. Beim Naphtha Reforming stellt die Oktanzahl diesen Zielparameter dar.

Das Erreichen dieses Zielparameters erfolgt beispielsweise durch eine Änderung der Prozessparameter wie Temperatur, Druck, WHSV, etc. Insbesondere beim Betrieb des erfindungsgemäßen Verfahrens in einer parallelen Anordnung, bei der eine Vielzahl von Katalysatoren gleichzeitig und parallel untersucht wird, ergeben sich Vorteile in Verbindung mit dem erfindungsgemäßen Verfahren. In einer bevorzugten Ausführungsform betrifft das Verfahren auch das Einregeln des Zielparameters auf einen festen Wert beziehungsweise in Verbindung mit einer Vorrichtung mit einer Vielzahl von parallel angeordneten Reaktoren, das jeweilige Einregeln der Zielparameter in den parallel durchgeführten Prozessen. In denjenigen Fällen, in denen eine Deaktivierung des Katalysators auftritt, betrifft das Verfahren auch das Nachregeln des Prozesses, um dem Zielparameter - trotz des Auftretens einer Deaktivierung - zu erreichen und zu halten.

In Verbindung mit dem Verfahren ist es von Bedeutung, dass das Ein- und Nachregeln schneller erfolgt als die zeitliche Änderung des Produktspektrums und die Deaktivierung der Katalysatoren. Ein Vorteil des erfindungsgemäßen Verfahrens ist auch, dass die Durchführung der online-IR-Messungen während der Messphase eine genaue und schnelle Analyse ermöglicht und somit auch eine schnelle Nachregelung gewährleistet.

Im vorliegenden Beispiel wurde das erfindungsgemäße Verfahren in Verbindung mit einem katalytischen Verfahren zur Umwandlung und Veredelung von einem Aromatengemisch aufgezeigt. Bei dem eingesetzten Aromatengemisch handelte es sich um ein Gemisch aus einkernigen Aromaten, die unterschiedliche Anzahlen von Alkylsubstituenten und Positionen der Alkylsubstituenten aufwiesen. Das Ziel des Verfahrens war es, ein Produkt mit einem hohen Anteil an p-Xylol-herzustellen. Das Verfahren wurde in der Weise durchgeführt, dass die Konversion an Aromaten als Zielparameter ausgewählt wurde. Die Untersuchungen wurden mittels einer Apparatur zur Testung von Katalysator durchgeführt, die mit vier parallel angeordneten Reaktoren ausgestattet war. Jeder der vier parallel angeordneten Reaktoren wurde mit einem anderen Katalysatormaterial befüllt, die nachfolgend Katalysator K1 - K4 genannt werden.

In einer ersten Verfahrensvariante erfolgte die Analyse des Prozessstroms ausschließlich mit einem online-Gaschromatograph (1), wobei die Spektren automatisiert ausgewertet und die Konversion bestimmt wurden. Die Dauer der einzelnen gaschromatographischen Analyse lag bei ca. 30 min. Um die Prozessströme der vier parallel angeordneten Reaktoren mittels des einen online-Gaschromatographen (1) in sequentieller Weise zu charakterisieren, war eine Zeitdauer von 2 h erforderlich, um einen Messwert pro Prozessstrom zu erhalten.

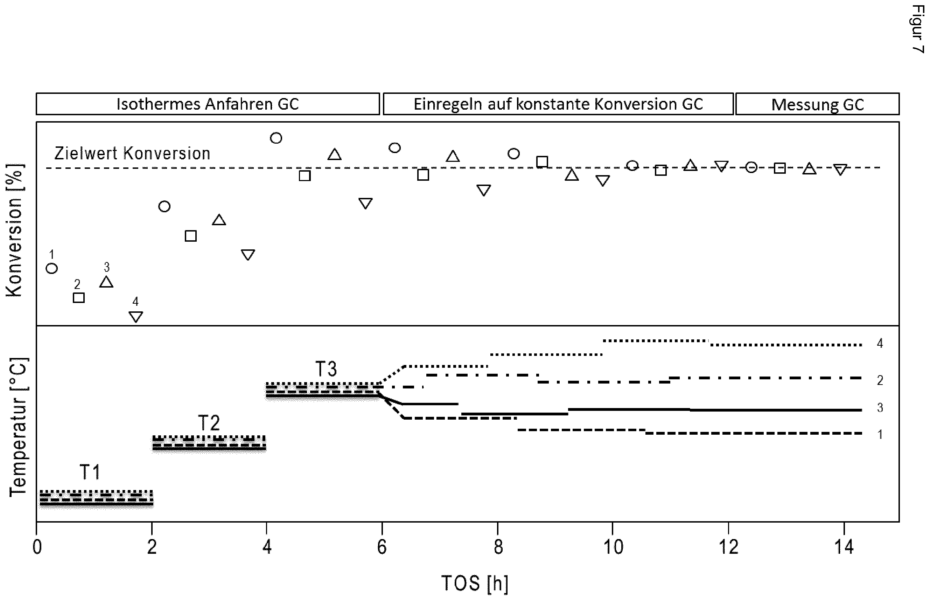

Die vier parallel angeordneten Reaktoren mit den Katalysatoren K1 bis K4 wurden zunächst isotherm bei gleicher Temperatur angefahren, wobei initiale Information über den Zusammenhang der Konversion mit der Temperatur gewonnen wurden. Vertieft wurden diese Messungen, die bei gleicher Temperatur durchgeführt worden waren, durch zusätzliche Messungen bei unterschiedlichen Temperaturen, um dadurch eine Kalibrierfunktion zu erhalten, die die Relation zwischen der Konversion und der Temperatur zeigte. Die Ergebnisse der Untersuchung sind in der Figur 7 dargestellt, wobei im unteren Teil die Temperatur als Funktion der TOS (TOS = Time On Stream) und im oberen Teil die Konversion als Funktion der TOS gezeigt werden. Ab TOS von 6 Stunden beginnt unter Einbeziehung dieser Kalibrierfunktion das Einregeln auf einen Zielwert der Konversion, wobei das Ergebnis aus der gaschromatischen Analyse automatisiert an die Prozesskontrolle übermittelt und die Temperatur angepasst wird. Es ist ersichtlich, dass nach etwas 3 Zyklen mit jeweils 2 h Länge dieselbe Konversion erreicht ist und die eigentliche Messung der Katalysatoren bei gleicher Konversion erfolgen kann. Im vorliegenden Fall hatte der Einregelprozess etwa 6 Stunden benötigt. Die Durchführung des Verfahrens mit einer Vorrichtung, die einen höheren Parallelisierungsgrad aufweist und die beispielsweise mit 16 Reaktoren ausgestattet ist, würde dies dann in der entsprechenden Weise 16 x 30 min Messzeit pro Zyklus erfordern, was bei drei Zyklen zu einer Dauer von 24 Stunden führen würde.

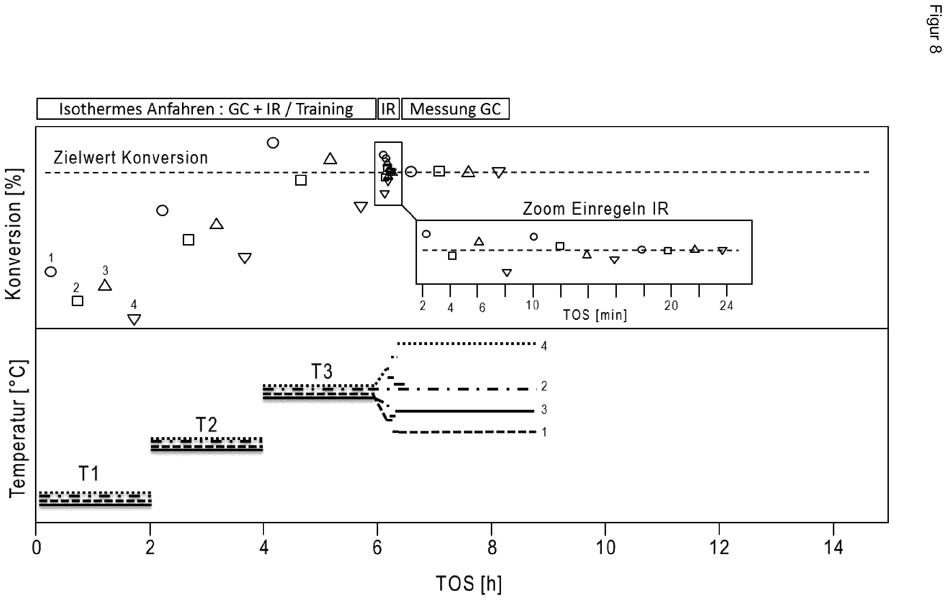

Untersuchungen zur Durchführung des Verfahrens in einer zweiten Verfahrensvariante werden im Folgenden beschrieben. In Figur 8 ist das erfindungsgemäße Verfahren unter Einbeziehen eines online-IR-Spektrometers (2) gezeigt. Die anfängliche Phase, bei der die Reaktoren isotherm betrieben wurden, wurde zum Aufnehmen der Kalibrierfunktion verwendet. Der online-Gaschromatograph (1) und das online-IR-Spektrometer (2) wurden parallel betrieben. Dabei wurden GC-Chromatogramme mit der Information zur Konversion und die zugehörigen IR-Spektren erhalten. Zugehörige IR-Spektren bedeutet, dass diese zeitgleich mit den GC-Chromatogrammen aufgenommen wurden. Anhand der erhaltenen Messdaten, die durch die GC-Chromatogramme und IR-Spektren als Funktion der Zeit gegeben sind, konnte mittels PLS ein Modell trainiert werden. Im Anschluss daran konnte beim Einregeln auf die konstante Konversion auf die Aufnahme von GC-Chromatogrammen verzichtet werden. Die Untersuchung der Prozessströme erfolgte auf der Basis der Aufnahme von online-IR-Spektren unter Bestimmung der Konversion mithilfe des vorher erstellten Models. Die Messzeit konnte in diesem Beispiel von 30 Minuten auf 2 Minuten reduziert werden und die Zielkonversion wurde innerhalb 24 min erreicht. In diesem Beispiel zeigte das erfindungsgemäße Verfahren einen signifikanten Geschwindigkeitsvorteil gegenüber einem Verfahren, das ohne die Kombination mit einem online-IR-Spektrometer durchgeführt wurde. Um Messpunkte mit höherer Informationsdichte zu erhalten, wurde der online-Gaschromatograph (1) beim Erreichen einer konstanten Zielkonversion zugeschaltet. Die zusätzliche Verwendung des online-Gaschromatograph (1) zur Charakterisierung der Prozessströme hatte den Vorteil, dass die Bestimmung der Selektivitäten weiter verbessert werden konnte. Die online-IR-Spektroskopie lieferte den Regelparameter, wobei vorliegend die Konversion als Regelparameter verwendet wurde. Eine verbesserte Bestimmung der Selektivitäten ermöglichte es, die Differenzierung der Katalysatoren zu verbessern.

Das vorliegend beispielhaft beschriebene Verfahren zur Veredelung von Aromatengemischen kann in der gleichen Weise als Verfahren für Transalkylierungsreaktionen, zur Dealkylierung von Ethylbenzol, zur Disproportionierung von Toluol, zur Isomerisierung von Xylolen und in anderen Verfahren eingesetzt werden.

Kurze Beschreibung der Figuren:

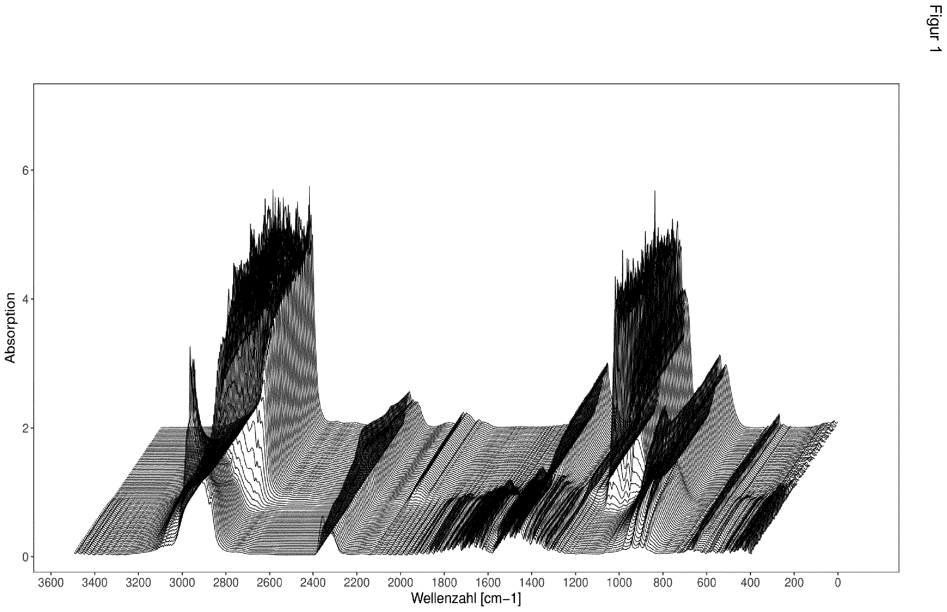

- Figur 1: zeigt eine Serie von MIR-Spektren, die während der Trainingsphase des Verfahrens bei einer WHSV von 0,4 h⁻¹ aufgenommen wurde. Der Verfahrensschritt ist in der Tabelle 1 mit der Nummer Experiment C1_03 gekennzeichnet. Die Zeitabstände für die Aufzeichnung der Spektren lagen bei 5 Sekunden, wobei die Spektren gezeigt sind, die über den Zeitraum von mehreren Minuten aufgezeichnet worden sind.

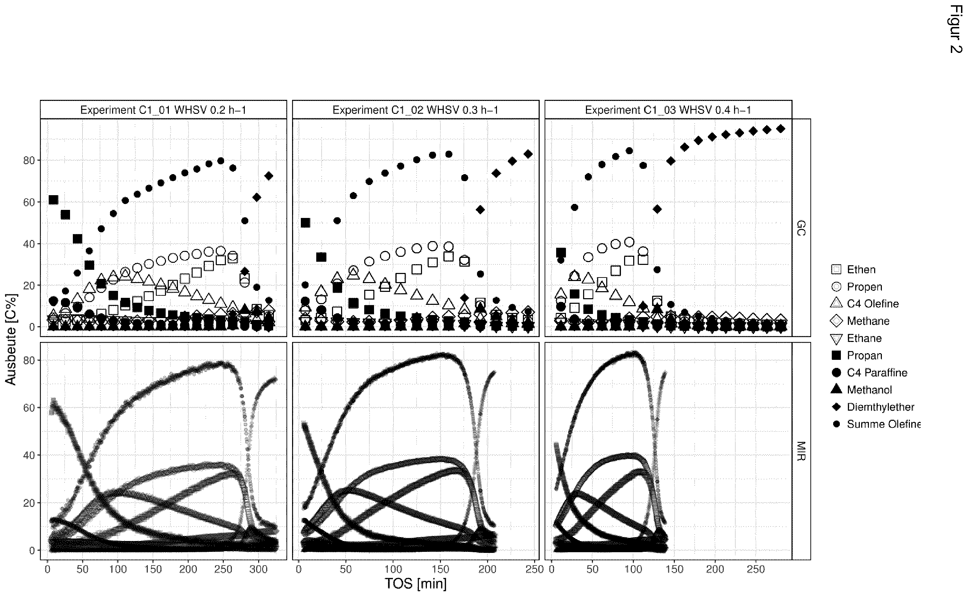

- Figur 2: zeigt die Zusammensetzung des Prozessstromes in Abhängigkeit von der Zeit, die während der Trainingsphase mittels GC bei drei unterschiedlichen Gaslasten bestimmt worden waren und die für MIR-Spektren berechnet wurden. Die WHSV's lagen bei 0,2 h⁻¹, 0,3 h⁻¹ und 0,4 h⁻¹ und wurden bei den Experimenten mit den Versuchsnummern C1_01, C1_02 und C1_03 aufgenommen. Figur 2 oberes Panel: Ausbeuten der gaschromatographischen Analyse; Figur 2 unteres Panel: vorhergesagte Ausbeuten der MIR-Spektren.

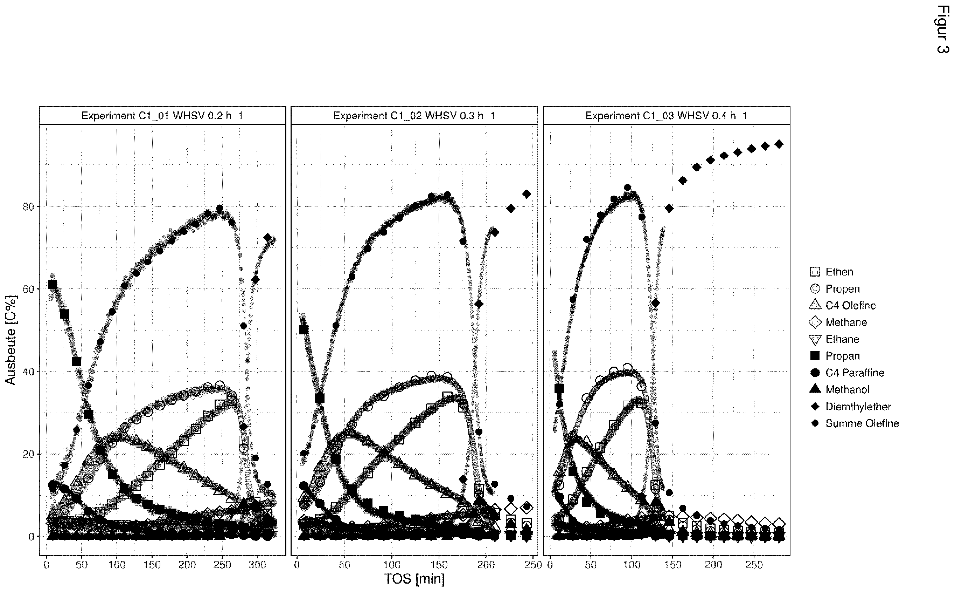

- Figur 3: zeigt Daten die während der Trainingsphase bei niedrigen WHSV`s erzielt wurden, und zwar in einer überlagerten Darstellung von GC- und MIR-Daten. Die großen Symbole charakterisieren die Ausbeuten, die mittels gaschromatographischen Analysen ermittelt wurden; die kleinen Symbole charakterisieren, die vorhergesagten Ausbeuten, die anhand der MIR-Spektren bestimmt wurden.

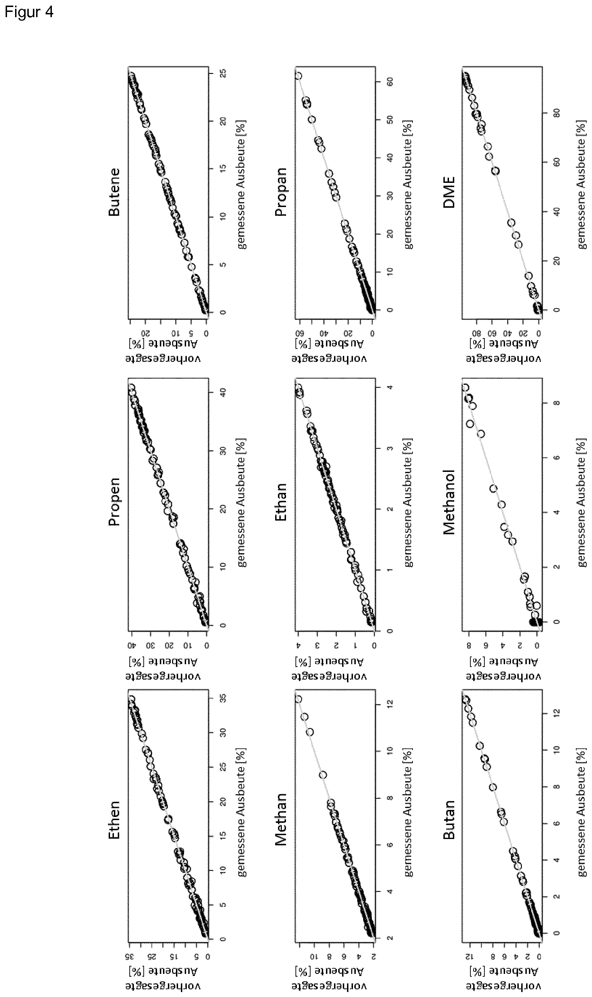

- Figur 4: zeigt die Modelle, welche durch das Inbeziehungsetzen von GC-Daten und IR-Daten während der Trainingsphase erhalten wurden. Es werden für 9 unterschiedliche Einzelsubstanzen die vorhergesagten Ausbeuten gegenüber den gemessenen Ausbeuten dargestellt.

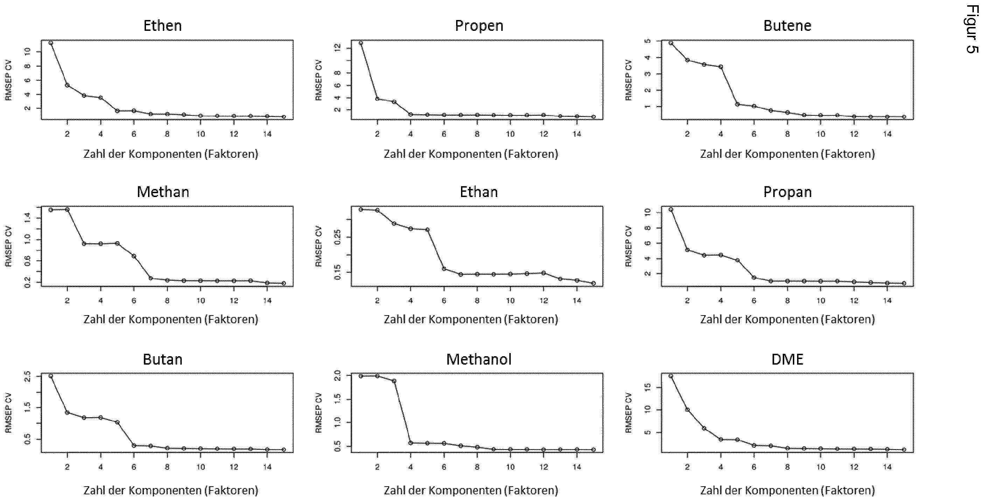

- Figur 5: zeigt den RMSEP (root mean square error predicted) der Kreuzvalidierung für die trainierten Modelle der 9 unterschiedlichen Einzelsubstanzen gegen die Zahl der Komponenten aufgetragen, wobei bis zu 15 Komponenten verwendet wurden.

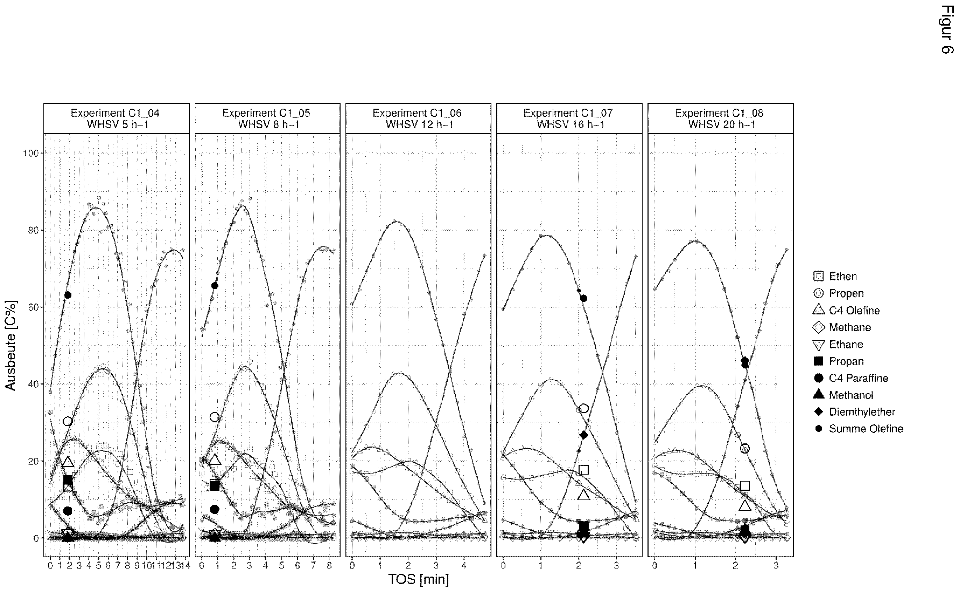

- Figur 6: zeigt die während der Produktionsphase vorhergesagten Ausbeuten der MIR-Spektren bei WHSV's, die im Zielparameterraum lagen (d.h. hohe WHSV's waren in fünf Experimenten bei 5, 8, 12, 16 und 20 h⁻¹). Die großen Symbole: repräsentieren Ausbeuten der gaschromatographischen Analyse, die kleinen Symbole: repräsentieren vorhergesagte Ausbeuten des MIR.)
- Figur 7: zeigt Werte für Konversion und Temperatur als Funktion der Time-on-Stream (TOS), die bei der Durchführung eines Verfahrens zur Konversion von Aromaten erhalten wurden, bei dem 4 Katalysatoren in einem 4-fach parallelisierten Reaktorsystem angeordnet waren. Oben die Konversion, unten die Reaktortemperatur. Die Konversion wurde ausschließlich mit Gaschromatograph bestimmt.
- Figur 8: zeigt Werte für Konversion und Temperatur als Funktion der Time-on-Stream (TOS), die bei der Durchführung erfindungsgemäßen Verfahrens erzielt wurden, bei dem vier Katalysatoren in vier parallel angeordneten Reaktoren untersucht wurden. Oben die Konversion, unten die Reaktortemperatur. Die Konversion wurde mit online-Gaschromatograph (1) bestimmt, das Einregeln auf die konstante Konversion mit online-IR-Spektrometer (2) durchgeführt.
- Figur 9: zeigt eine schematische Darstellung der erfindungsgemäßen Vorrichtung in einer Ausführungsform, die mit fünf parallel angeordneten Reaktionsräumen (11) - (15) ausgestattet ist, wobei das online-Spektrometer (2) und der online-Gaschromatograph (1) seriell in der Prozessstromleitung (35) angeordnet sind, und die Prozesssteuereinheit (4) mit den Reaktionsräumen gekoppelt ist.
- Figur 10: zeigt eine schematische Darstellung der erfindungsgemäße Vorrichtung in einer Ausführungsform, die der in Figur 9 gezeigten Ausführungsform entspricht, wobei die Prozessstromleitung (35) in zwei Leitungen aufgeteilt vorliegt und das online-IR (2) und der online-GC (1) in diesen zwei Leitungen parallel angeordnet sind.

**Bezugszeichenliste**

| | | |
|---|---|---|
| 1 | - | online-GC, online-Gaschromatograph |
| 2 | - | online-IR, online-IR-Spektormeter bzw. online-Spektrometer, vorzugweise optimiert für den MIR-Bereich |
| 4 | - | Prozesssteuereinheit |
| 35 | - | Prozessstromleitung |
| 34 | - | Abluftleitung |
| 33 | - | Multiportventil |
| 21, 22, - 25 | - | reaktionsraumausgangsseitige Prozessgasleitungen in Verbindung mit Multiportventil |
| 11, 12, - 15 | - | Prozessräume, vorzugsweise Reaktionsräume, weiter vorzugsweise Rohrreaktoren |

## Patentansprüche

1. Verfahren zur Untersuchung von zumindest einem aus einem katalytischen Prozess stammenden Prozessstrom, der zumindest fünf verschiedene kohlenwasserstoffhaltige Komponenten umfasst, wobei das Verfahren zumindest die folgenden Schritte umfasst:
a) Bereitstellen von zumindest einer Prozessstromleitung (35), welche in Wirkverbindung mit zumindest einem online-IR-Spektrometer (2), sowie in Wirkverbindung mit zumindest einem online-Gaschromatographen (1) ist;
b) Durchleiten des zumindest einen Prozessstroms durch die zumindest eine Prozessstromleitung (35), wobei während dieses Durchleitens des Prozessstroms durch die Prozessstromleitung (35) eine analytische Charakterisierung des Prozessstroms mittels online-IR-Spektrometer (2) und online-Gaschromatograph (1) durchgeführt wird;
c) Auswerten der bei der analytischen Charakterisierung des Prozessstroms mittels online-IR-Spektrometer (2) erhaltenen Spektral-Daten als Funktion eines Durchführungszeitpunkts dieser spektroskopischen Analyse des Prozessstroms,
d) Auswerten der bei der analytischen Charakterisierung des Prozessstroms mittels des online-Gaschromatographen (1) erhaltenen Chromatographie-Daten als Funktion einer Probenentnahmezeit für dabei aus dem Prozessstrom entnommene Proben,
e) Auf maschinellem Lernen beruhendes Trainieren eines Modells, das eine mathematischen Beziehung zwischen Spektral-Daten und dazu korrespondierenden Chromatographie-Daten bezüglich eines selben Prozessstroms modelliert, mittels der in den Schritten c) und d) erhaltenen Auswertungsergebnisse bezüglich des in Schritt b) durch die Prozessstromleitung geleiteten Prozessstroms.

2. Verfahren zur Untersuchung von zumindest einem aus einem katalytischen Prozess stammenden Prozessstrom nach Anspruch 1, wobei das Verfahren eine Rückkopplung umfasst und wobei der online-Gaschromatograph (1) und das online-IR-Spektrometer (2) mit einer gemeinsamen Prozesssteuereinheit (4) gekoppelt sind, wobei die gemeinsame Prozesssteuereinheit (4) mit einem Prozessraum (11) in Wirkverbindung steht und den im Prozessraum ablaufenden Prozess kontrolliert, steuert oder regelt.

3. Verfahren nach Anspruch 1 oder Anspruch 2, wobei die Schritte c) - e) durchgeführt werden und zumindest ein Reaktionsparameter gegenüber demselben Reaktionsparameter, wie dieser in den Schritten a) und/oder b) eingestellt wird, verändert ist, wobei dieser Parameter ausgewählt ist aus: WHSV, Temperatur, Gesamtdruck und/oder Partialdruck von Reaktanden.

4. Verfahren zur Untersuchung von zumindest einem Prozessstrom nach Anspruch 1 bis Anspruch 3, das **dadurch gekennzeichnet ist, dass** es zumindest zwei unterschiedliche Phasen umfasst, wobei es sich bei der einen Phase um eine die Schritte a) bis e) umfassende Trainingsphase und bei der zweiten Phase um eine eigentliche Messphase handelt, in der eine analytische Charakterisierung eines durch die zumindest eine Prozessstromleitung geleiteten Prozessstroms mittels des online-IR-Spektrometers (2) auf Basis des in der Trainingsphase trainierten Modells erfolgt.

5. Verfahren zur Untersuchung von zumindest einem Prozessstrom nach zumindest einem der vorstehenden Ansprüche, das **dadurch gekennzeichnet ist, dass** es sich bei dem durch die Prozessstromleitung (35) geführten Prozessstrom um einen gasförmigen Prozessstrom handelt, vorzugsweise ist die Temperatur des gasförmigen Prozessstroms im Bereich von 20 - 350 ° C.

6. Verfahren zur Untersuchung von zumindest einem Prozessstrom nach einem der Ansprüche 1 - 5, das **dadurch gekennzeichnet ist, dass** der online-Gaschromatograph (1) und das online-IR-Spektrometer (2) serielle Anordnung in Bezug auf die Prozessstromleitung aufweisen und der zeitliche Versatz von 1 sec bis 180 sec beträgt.

7. Verfahren zur Untersuchung von zumindest einem Prozessstrom nach einem der Ansprüche 1 - 6, das **dadurch gekennzeichnet ist, dass** die Analyseneinheiten, online-Gaschromatograph (1) und das online-IR-Spektrometer (2), mit einer gemeinsamen Prozesssteuereinheit (4) gekoppelt sind, wobei die Prozesssteuereinheit mit einem Prozessraum (11) in Wirkverbindung steht und den im Prozessraum ablaufenden Prozess kontrolliert, steuert oder regelt.

8. Verfahren zur Untersuchung von zumindest einem Prozessstrom nach Anspruch 7, wobei die Prozesssteuereinheit (4) den Prozess so regelt, dass mittels der Anpassung eines Prozessbetriebsparameters die Produktstruktur kontrolliert wird.

9. Verfahren zur Untersuchung von zumindest einem Prozessstrom nach einem der Ansprüche 1 bis 8, wobei der Prozess zumindest einen Prozess aus der Gruppe ausgewählt aus Methanol-Konversionsprozesse wie MTO (Methanol-to-Olefins), Dehydrierreaktionen wie Propandehydrogenierung, Kopplungsreaktionen wie Methankopplung, Naphtha Reforming und Prozesse zur Umwandlung von Aromaten umfasst.

10. Verfahren zur Untersuchung von zumindest einem Prozessstrom nach einem der Ansprüche 1 - 9, wobei das online-IR-Spektrometer (2) im mittleren IR Bereich (MIR) arbeitet und die Wellenzahlen im Bereich von 400 cm⁻¹ - 3500 cm⁻¹ liegen.

11. Verfahren zur Untersuchung von zumindest einem Prozessstrom nach einem der Ansprüche 1 - 10, wobei das Modell oder die Methode wie im Schritt e) eingesetzt, eine statistische Methode umfasst, die ausgewählt ist aus der Gruppe multivariater Analysen wie Hauptkomponentenanalyse (PCA), partieller Regression kleinster Quadrate (PLS), Hauptkomponentenregression (PCR), multilinearer Regressionsanalyse (MLR), Diskriminanzanalyse oder neuronaler Netze.

12. Verfahren zur Untersuchung von zumindest einem Prozessstrom nach einem der Ansprüche 1 - 11, wobei zusätzliche Daten zum Training des Modells, welche gemäß den Schritten c) und e) vorgesehen sind, durch eine Variation von Prozessparametern wie der Temperatur, des Drucks, des Partialdrucks der Edukte oder der WHSV erhalten werden.

13. Verfahren zur Untersuchung von zumindest einem Prozessstrom nach einem der Ansprüche 1 - 12, wobei die Masse des für den Prozess eingesetzten Katalysators im Bereich 0,1-200 ccm.

14. Verwendung des Verfahrens nach einem der vorstehenden Ansprüche 1 bis 13 in der Hochdurchsatztestung von zumindest vier, vorzugsweise zumindest acht, weiter vorzugsweise zumindest zwölf, in parallelen Reaktoren angeordneten Katalysatoren.

## Claims

1. A method for investigating at least one process stream deriving from a catalytic process and comprising at least five different hydrocarbon-containing components, wherein the method comprises at least the steps of:
a) providing at least one process stream conduit (35) which is in operative connection with at least one online IR spectrometer (2) and in operative connection with at least one online gas chromatograph (1);
b) passing the at least one process stream through the at least one process stream conduit (35), wherein during this passing of the process stream through the process stream conduit (35) an analytical characterization of the process stream using an online IR spectrometer (2) and an online gas chromatograph (1) is performed;
c) evaluating the spectral data obtained in the analytical characterization of the process stream using an online IR spectrometer (2) as a function of the time at which this spectroscopic analysis of the process stream was carried out,
d) evaluating the chromatography data obtained during the analytical characterization of the process stream using the online gas chromatograph (1) as a function of a sampling time for samples taken from the process stream,
e) machine learning-based training of a model that models a mathematical relationship between spectral data and corresponding chromatography data in respect of an identical process stream by using the evaluation results obtained in steps c) and d) in respect of the process stream passed through the process stream conduit in step b) .

2. The method for investigating at least one process stream derived from a catalytic process according to claim 1, wherein the method comprises a feedback and wherein the online gas chromatograph (1) and the online IR spectrometer (2) are coupled to a common process control unit (4), wherein the common process control unit (4) is in operative connection with a process space (11) and controls, governs or regulates the process proceeding in the process space.

3. The method according to claim 1 or claim 2, wherein steps c) - e) are performed and at least one reaction parameter is altered in relation to the same reaction parameter as set in steps a) and/or b), wherein this parameter is selected from: WHSV, temperature, total pressure and/or partial pressure of reactants.

4. The method for investigating at least one process stream according to claim 1 to claim 3, wherein it comprises at least two different phases, wherein one phase is a training phase comprising steps a) to e) and the second phase is an actual measurement phase in which an analytical characterization of a process stream passed through the at least one process stream conduit is carried out using the online IR spectrometer (2) on the basis of the model trained in the training phase.

5. The method for investigating at least one process stream according to at least one of the preceding claims, wherein the process stream passed through the process stream conduit (35) is a gaseous process stream, the temperature of the gaseous process stream preferably being in the range of 20 - 350°C.

6. The method for investigating at least one process stream according to any of claims 1-5, wherein the online gas chromatograph (1) and the online IR spectrometer (2) are serially arranged in respect of the process stream conduit and the temporal offset is in the range of 1 sec to 180 sec.

7. The method for investigating at least one process stream according to any of claims 1-6, wherein the analysis units, the online gas chromatograph (1) and the online IR spectrometer (2), are coupled to a common process control unit (4), wherein the process control unit is in operative connection with a process space (11) and controls, governs or regulates the process proceeding in the process space.

8. The method for investigating at least one process stream according to claim 7, wherein the process control unit (4) regulates the process such that the product structure is controlled by adapting a process operating parameter.

9. The method for investigating at least one process stream according to any of claims 1 to 8, wherein the process comprises at least one process from the group selected from methanol conversion processes such as MTO (methanol to olefins), dehydrogenation reactions such as propane dehydrogenation, coupling reactions such as methane coupling, naphtha reforming and processes for conversion of aromatics.

10. The method for investigating at least one process stream according to any of claims 1 - 9, wherein the online IR spectrometer (2) operates in the mid IR range (MIR) and the wavenumbers are in the range of 400 cm⁻¹ - 3500 cm⁻¹.

11. The method for investigating at least one process stream according to any of claims 1 - 10, wherein the model or the method as employed in step e) comprises a statistical method selected from the group of multivariate analyses such as principal component analysis (PCA), partial least squares (PLS) regression, principal component regression (PCR), multi-linear regression (MLR) analysis, discriminant analysis or neural networks.

12. The method for investigating at least one process stream according to any of claims 1 - 11, wherein additional data for training the model, provided for according to steps c) and e), are obtained by variation of process parameters such as temperature, pressure, partial pressure of the reactants or WHSV.

13. The method for investigating at least one process stream according to any of claims 1 - 12, wherein the mass of the catalyst employed for the process is in the range of 0.1-200 ccm.

14. The use of the method according to any of the preceding claims 1 to 13 in high-throughput testing of at least four, preferably at least eight, more preferably at least 12, catalysts arranged in parallel reactors.

## Revendications

1. Procédé d'analyse d'au moins un flux de procédé provenant d'un procédé catalytique qui comprend au moins cinq composants hydrocarbonés différents, le procédé comprenant au moins les étapes suivantes :
a) mise à disposition d'au moins une conduite (35) à flux de procédé, qui est en liaison active avec au moins un spectromètre IR (2) en ligne ainsi qu'en liaison active avec au moins un chromatographe gazeux (1) en ligne ;
b) passage dudit au moins un flux de procédé à travers ladite au moins une conduite (35) à flux de procédé, une caractérisation analytique du flux de procédé pendant ce passage du flux de procédé à travers la conduite (35) à flux de procédé étant réalisée au moyen du spectromètre IR (2) en ligne et du chromatographe gazeux (1) en ligne ;
c) évaluation des données spectrales obtenues lors de la caractérisation analytique du flux de procédé au moyen du spectromètre IR (2) en ligne en fonction d'un moment de réalisation de cette analyse spectroscopique du flux de procédé,
d) évaluation des données de chromatographie obtenues lors de la caractérisation analytique du flux de procédé au moyen du chromatographe gazeux (1) en ligne en fonction d'un moment de prélèvement d'échantillon pour des échantillons prélevés du flux de procédé,
e) entraînement reposant sur un apprentissage machine d'un modèle, qui modélise une relation mathématique entre des données spectrales et des données chromatographiques correspondant à celles-ci concernant un même flux de procédé au moyen des résultats d'évaluation obtenus dans les étapes c) et d) concernant le flux de procédé guidé dans l'étape b) à travers la conduite à flux de procédé.

2. Procédé d'analyse d'au moins un flux de procédé provenant d'un procédé catalytique selon la revendication 1, le procédé comprenant un couplage en retour et le chromatographe gazeux (1) en ligne et le spectromètre IR (2) en ligne étant couplés à une unité de commande de procédé (4) commune, l'unité de commande de procédé (4) commune étant en liaison active avec un espace de procédé (11) et régulant, commandant ou réglant le procédé se déroulant dans l'espace de procédé.

3. Procédé selon la revendication 1 ou la revendication 2, les étapes c) - e) étant réalisées et au moins un paramètre de réaction étant modifié par rapport au même paramètre de réaction tel que celui-ci est réglé dans les étapes a) et/ou b), ce paramètre étant choisi parmi : WHSV (Weight hourly space velocity), température, pression totale et/ou pression partielle de réactifs.

4. Procédé d'analyse d'au moins un flux de procédé selon la revendication 1 à la revendication 3, **caractérisé en ce qu'**il comprend au moins deux phases différentes, l'une phase étant une phase d'entraînement comprenant les étapes a) à e) et la deuxième phase étant une phase de mesure en soi, dans laquelle une caractérisation analytique d'un flux de procédé guidé à travers au moins une conduite à flux de procédé est effectuée au moyen du spectromètre IR (2) en ligne sur la base du modèle entraîné dans la phase d'entraînement.

5. Procédé d'analyse d'au moins un flux de procédé selon au moins l'une des revendications précédentes, **caractérisé en ce qu'**il s'agit, pour le flux de procédé guidé à travers la conduite (35) à flux de procédé, d'un flux de procédé gazeux, la température du flux de procédé gazeux étant de préférence située dans la plage de 20-350°C.

6. Procédé d'analyse d'au moins un flux de procédé selon l'une des revendications 1-5, **caractérisé en ce que** le chromatographe gazeux (1) en ligne et le spectromètre IR (2) en ligne présentent un agencement en série par rapport à la conduite à flux de procédé et le décalage temporel représentant 1 s à 180 s.

7. Procédé d'analyse d'au moins un flux de procédé selon l'une des revendications 1-6, **caractérisé en ce que** les unités d'analyse, le chromatographe gazeux (1) en ligne et le spectromètre IR (2) en ligne sont couplés à une unité de commande de procédé (4) commune, l'unité de commande de procédé étant en liaison active avec un espace de procédé (11) et régulant, commandant ou réglant le procédé se déroulant dans l'espace de procédé.

8. Procédé d'analyse d'au moins un flux de procédé selon la revendication 7, l'unité de commande de procédé (4) réglant le procédé de manière telle que la structure de produit est régulée par l'adaptation d'un paramètre de fonctionnement du procédé.

9. Procédé d'analyse d'au moins un flux de procédé selon l'une des revendications 1 à 8, le procédé comprenant au moins un procédé du groupe choisi parmi les procédés de conversion de méthanol, tels que le MTO (méthanol à oléfine), les réactions de déshydratation telles que la déshydrogénation de propane, les réactions de couplage telles que le couplage de méthane, le reformage de naphta et les procédés de conversion d'aromatiques.

10. Procédé d'analyse d'au moins un flux de procédé selon l'une des revendications 1 - 9, le spectromètre IR (2) en ligne travaillant dans une zone d'IR moyens (MIR) et les nombres d'onde se situant dans la plage de 400 cm⁻¹ - 3500 cm⁻¹.

11. Procédé d'analyse d'au moins un flux de procédé selon l'une des revendications 1-10, le modèle ou la méthode utilisée comme dans l'étape e) comprenant une méthode statistique qui est choisie dans le groupe des analyses multivariées, telles que l'analyse en composantes principales (PCA), la régression des moindres carrés partiels (PLS), la régression sur composantes principales (PCR), l'analyse par régression linéaire multiple (MLR), l'analyse discriminante ou les réseaux neuronaux.

12. Procédé d'analyse d'au moins un flux de procédé selon l'une des revendications 1-11, des données supplémentaires pour l'entraînement du modèle, qui sont prévues selon les étapes c) et e), étant obtenues par variation de paramètres de procédé tels que la température, la pression, la pression partielle des produits de départ ou la WHSV.

13. Procédé d'analyse d'au moins un flux de procédé selon l'une des revendications 1-12, la masse du catalyseur utilisé pour le procédé étant située dans la plage de 0,1-200 cm³.

14. Utilisation du procédé selon l'une des revendications précédentes 1 à 13 dans le test à débit élevé d'au moins quatre, de préférence au moins huit, plus préférablement au moins douze catalyseurs agencés dans des réacteurs parallèles.
